# EUROPEAN PATENT SPECIFICATION

(11) **EP 0 910 331 B1**
(45) Date of publication and mention of the grant of the patent: **08.09.2004**
(21) Application number: 97924518.0
(22) Date of filing: 25.04.1997
(51) Int. Cl.: A61K 7/075, A61K 7/48, C07C 59/245

(54) **OXA DIACIDS AND RELATED COMPOUNDS FOR TREATING SKIN CONDITIONS**
OXADISÄUREN UND VERWANDTE VERBINDUNGEN ZUR BEHANDLUNG VON HAUTSTÖRUNGEN
OXADIACIDES ET COMPOSES VOISINS POUR TRAITER LES PROBLEMES DE PEAU

(30) Priority: 25.04.1996 US 636540
(43) Date of publication of application: 28.04.1999
(73) Proprietor: AVON PRODUCTS, INC., New York, NY 10020-1196 (US)
(72) Inventor: PTCHELINTSEV, Dmitri, Mahwah, NJ 07430 (US); SCANCARELLA, Neil, Wyckoff, NJ 07481 (US); KALAFSKY, Robert, Ogdensburg, NJ 07439 (US)
(74) Representative: Dr. Weitzel & Partner
(86) International application number: PCT/US1997/006973
(87) International publication number: WO 1997/039726

(56) References cited:
- EP-A- 0 413 528
- US-A- 4 136 098
- US-A- 4 721 579
- US-A- 5 108 751
- US-A- 5 464 929

## Description

### BACKGROUND OF THE INVENTION

### I. Field of Invention

The present invention relates to topical compositions comprising a compoundof formula (I) as defined below as active principals for topical treatment of skin conditions, to the use of this compound for the manufacture of a medicament and to a method of peeling skin or softening the hair

Compounds of the class include those of Formula (I): 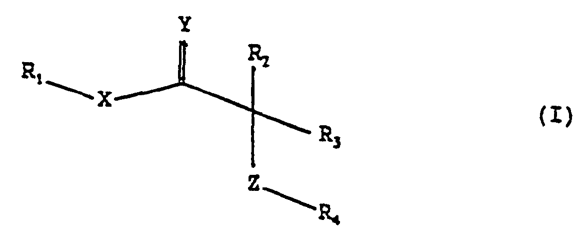 wherein, R₄ is (CR₅R₆-CR₇R₈-X₁)ₙ-CR₉R₁₀-C (=X₂) X₃R₁₁; n is an integer from 1 to 18; R₁, R₂, R₃, R₅, R₆, R₇, R₈, R₉, R₁₀ and R₁₁, are independently hydrogen or non-hydrogen substituents, with preferred non-hydrogen substituents including alkyls, alkenyls, oxa-alkyls, aralkyls and aryls; and X, X₁, X₂, X₃, X₃, Y and Z are independently, 0, NH or S, with preferred compounds including those in which X, X₁, X₂, X₃, Y and Z are each oxygen and R₁, R₂, R₃, R₅, R₆, R₇, R₈, R₉, R₁₀ and R₁₁ are each hydrogen.

### II. Description of the Prior Art

Dermal use of alpha hydroxyacids having an all carbon backbone is described in U.S. Patent No. 5,091,171. Cosmetic compositions using 2-hydroxyalkenoic acid are disclosed, for example, in U.S. Patent No. 5,108,751. Such compounds must have an unsubstituted alpha hydoxy group on a carbon backbone and are purportedly used to impart beneficial effects to the skin. However, the trend is away from the use of such alpha hydroxyacids since they necessitate low operational pH ranges that for the most common forms, i.e. glycolic and lactic acids, are known to cause skin irritations.

Topical formulations comprising straight, all carbon backbone, dicarboxylic acids have been proposed as replacements for alpha hydroxyacids. For example, U.S. Patent Nos. 4,292,326, 4,386,104 and 5,385,938 describe the use of dicarboxylic acids having 7 to 13 carbon atoms for various skin indications. Similarly, U.S. Patent No. 4,885,282 states that a 4 to 18 carbon dicarboxylic acid compound is useful for the treatment of skin disorders.

The problem with the use of these dicarboxylic acids is their inherent insolubility in aqueous solutions. Such solutions make up the majority of cosmetic delivery systems. Also, dicarboxylic acids that have all carbon backbones are solid at ambient temperatures, extremely difficult to work with and, if a solution is achieved, the result is an aesthetically unpleasant mixture unsuitable for cosmetic use.

Therefore, there is a need for a compound or class of compounds that can be used as mild, exfoliating actives for topical treatment of skin.

There is also a need for a mild, exfoliating topical composition that contains a water soluble compound that can be manufactured into an aesthetically acceptable cosmetic or dermatologic products.

### SUMMARY OF THE INVENTION

It is an object of the present invention to indicate the use of a water soluble compound or class of such compounds that can be manufactured into an aesthetically acceptable, mild, exfoliating composition .

It is another object of the present invention to provide topical compositions with multiple skin care benefits.

It is a further object of the present invention to provide a new, dermatologic and cosmetic use for oxa diacids.

These and other objects will become evident from the following disclosure.

### DETAILED DESCRIPTION OF THE INVENTION

The basic compound used for this invention is a compound of the following Formula (I): 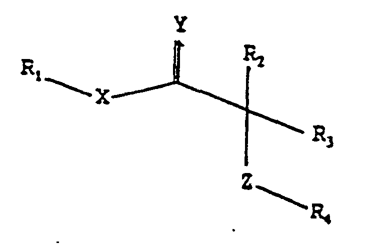 wherein, R₄ is (CR₅R₆-CR₇R₈-X₁)⁻ₙ-CR₉R₁₀-C(=X₂)X₃R₁₁; n is an integer from 1 to 18 preferably n is an integer from 2 to 12; R₁, R₂, R₃, R₅, R₆, R₇, R₈, R₉, R₁₀ and R₁₁ are independently hydrogen or non-hydrogen substituents.

X, X₁, X₂, X₃, Y and Z are independently, 0, NH, or S. Preferred are those compounds in which X, X₁, X₂, X₃, Y and Z are all Oxygen or each amino groups or each sulfur. Most preferred are those compounds in which X, X₁, X₂, X₃, Y and Z are each oxygen, and R₁, R₂, R₃, R₅, R₆, R₇, R₈, R₉, R₁₀ and R₁₁, are each hydrogen.

The preferred non-hydrogen substituents include alkyls alkenyls, oxa-alkyls, aralkyls and aryls. Examples of non-hydrogen substituents include methyl, ethyl, propyl, isopropyl, butyl, isobutyl, hexyl, heptyl, octyl, nonyl, dodecanyl, methoxy, ethoxy, propoxy, butoxy, cyclohexenyl, hydroxymethyl, hydroxyethyl, hydroxypropyl, cyclobutyl and cyclohexanyl.

Preferred compounds of Formula (I) include 3,6-dioxaoctadioic acid (HOOC-CH₂-O-CH₂-CH_{Z}-O-CH_{Z}-COOH) ; 3,6,9-trioxaundecane-dioic acid (HOOC-CH₂-O-CH₂-CH₂-O-CH₂-CH₂-O-CH₂-COOH); 3,6,9,12-tetraoxatetradecanedioic acid (HOOC-CH₂-O-CH₂-CH₂-O-CH₂-CH₂-O-CH₂-CH₂-O-CH₂-COOH) ; 3,6,9,12, 15-pentaoxa-heptadecanedioic acid (HOOC-CH₂-O-CH₂-CH₂-O-CH₂-CH₂-O-CH₂-CH₂-O-CH₂-CH₂-O-CH₂-COOH); 2-methyl-3,6,9-trioxaundecanedioic acid (HOOC-CH₂-O-CH₂-CH₂-O-CH₂-CH-O-CH (CH₃) -COOH); 2-ethyl-3,6,9,12-tetraoxatetradecane-dioic acid (HOOC-CH₂-O-CH₂-CH₂-O-CH₂-CH₂-O-CH₂-CH₂-O-CH (C₂H₅) -COOK) ; 2-phenyl-3,6,9-trioxaundecanedioic acid (HOOC-CH (Ar) -O-CR₂-CH₂-O-CH₂-CH₂-O-CH₂-COOH) ; 3,6,9-trioxaun-decanedioic acid diethyl ester (H₂C₂-OOC-CH₂-O-CH₂-CH₂-O-CH₂-CH₂-O-CH₂-COO-C₂H₅); 3,6,9-triaminoun-decanedioic acid (HOOC-CH₂-NH-CH₂-CH₂-NH-CH₂-CH₂-NH-CH₂-COOH); 3,6,9,12-tetraminotetradecanedioic acid (HOOC-CH₂-NH-CH₂-CH₂-NH-CH₂-CH₂-NH-CH₂-CH₂-NH-CH₂-COOH); 3-amino-6,9-dioxaundecanedioic acid (HOOC-CH₂-NH-CH₂-CH₂-O-CH₂-CH₂-O-CH₂-COOH); 3,6-diamino-9-oxaundecanedioic acid (HOOC-CH₂-NH-CH₂-CH₂-NH-CH₂-CH₂-O-CH₂-COOH) ; 3,6,9-trithioundecanedioic acid (HOOC-CH₂-S-CH₂-CH₂-S-CH₂-CH₂-S-CH₂-COOH) ; 3,6-dithio-9,12-dioxatetradecanedioic acid (HOOC-CH₂-S-CH₂-CH₂-S-CH₂-CH₂-O-CH₂-CH₂-O-CH₂-COOH) ; 3-amino-6,9-dioxaundecanedioic acid monoamide (HOOC-CH₂-NH-CH₂-CH₂-O-CH₂-CH₂-O-CH₂-CO-NH₂); 3-amino-6,9-dioxa-undecane-dioic acid diamide (H₂N-OC-CH₂-NH-CH₂-CH₂-O-CH₂-CH₂-O-CH₂-CONH₂) ; 3,6,9-trioxaundecanedioic acid monoamide (HOOC-CH₂-O-CH₂-CH₂-O-CH₂-CH₂-O-CH₂-CONH₂) ; 3,6,9-trioxaundecanedioic acid diamide (H₂NOC-CH₂-O-CH₂-CH₂-O-CH₂-CH₂-O-CH₂-CONH₂); 2-10-dimethyl-3,6,9-trioxa-undecanedioic acid (HOOC-CH-(CH₃)-O-CH₂-CH₂-O-CH₂-CH₂-O-CH (CH₃)-COOH); and 2, 10-dimethyl-3,9-dithio-6-oxaun-decanedioic acid (HOOC-CH(CH₃)-S-CH₂-CH₂-O-CH₂-CH₂-S-CH(CH₃)-COOH).

Compounds of Formula (I) are described as intermediates useful in the making of curing agents and hardeners for epoxy resins in U.S. Patent Nos. 5,017,675 and 5,319,004, both assigned to Hoechst AG. German Published Application No. DE-A-2936123 describes the preparation of such epoxy resin intermediate compounds. Such compounds are also commercially available from Hoechst AG.

Compounds of Formula I can also be prepared from commercially available polamines, polyols and polythiols by routine chemical reactions well known to those skilled in the art such as amidation, catalytic oxidation, esterification and other well known organic chemistry synthetic protocols, as described in organic chemistry textbooks including March, Advanced Organic Chemistry: Reactions, Mechanisms, and Structure, 3rd ed., John Wiley Interscience (1985) and Carey et al. Advanced Organic Chemistry, 3rd ed., Parts A and B, Plenum Press, New York (1990).

The oxa compounds useful in the topical compositions of this invention can also be in the form of derivatives that are converted back to an acidic form by action of hydrolytic enzymes in the skin such as glycosidases, phosphatases, esterases and amidases. Examples of suitable derivatives include esters of Formula I compounds with aliphatic alcohols, carbohydrates, amides, lactones and anhydrides

As defined herein, all compounds of Formula (I), and derivatives thereof, will be referred to collectively as "oxa diacids" and/or "oxa compounds" and/or "oxa diacid compounds".

A "topical application" refers to spreading or laying directly onto the surface of skin. A "topical composition" refers to a composition intended to be directly layed onto or spread on the surface of skin. An "effective amount" means an amount of a compound or a composition sufficient to induce a positive change (e.g. normalization of desquamation) in the skin condition to be treated such as those attributed to, accompanied or exacerbated by abnormal desquamation. A "physiologically acceptable vehicle" or a "suitable topical vehicle" refers to drugs, cosmetics, medicaments or inert ingredients that are suitable for use in direct contact with human tissues without undue toxicity. All percentages refer to weight percent, based on the total weight of the topical composition.

In accordance with the invention, oxa compounds are used as active principals in topical applications to treat various skin conditions attributed to, accompanied by or exacerbated by abnormal desquamation. Such conditions include, but are not limited to, dry skin, ichthyosis, palmar and plantar hyperkeratoses, dandruff, lichen simplex chronicus, Dariers disease, keratoses, lentigines, age spots, melasmas, blemished skin, acne, psoriasis, eczema, pruritis, inflammatory dermatoses, striae distensae (i.e. stretch marks), warts and calluses.

The compounds are unexpectedly and surprisingly found to be useful as active agents in topical preparations for treating signs of dermatological aging, both photoaging and intrinsic aging, including skin wrinkles such as fine wrinkling in the eye area or "crows feet" or fine wrinkles around the mouth area, irregular pigmentation, sallowness, loss of skin resilience and elasticity.

Oxa compounds and topical compositions containing them are also useful for treating disorders associated with the nails, cuticles and hair such as ingrown hair, folliculitis and Pseudofolliculitis barbae. The present compounds also soften hair and promote the elimination of hair ingrowths, making the compounds of Formula (I) useful in shaving compositions.

The oxa compounds can be incorporated into the compositions as free acids or as corresponding salts derived by neutralization with organic or inorganic bases such as triethanolamine, arginine, lysine, potassium hydroxide, sodium hydroxide, lithium hydroxide, ammonium hydroxide.

When used in combination with a physiologically acceptable vehicle to form a topical composition, the effective amount of the oxa compound can be within the range from about 0.1% to about 95%, preferably from about to about 50 wt % more preferably from 5 to about 20 wt %. Additionally, it is prefered that the topical composition comprises about 1 to about 50 wt % of the compound of Formula (I) and has a pH of less than 7.0. Beside the 0.1 to about 95 wt % of compound of formula (I) the topical composition preferably comprises 0.5 to about 50 wt % of an emollient and about 0.1 to about 30 wt % of an emulsifier. In such a case, said compound can be selected from the group consisting of 3,6-dioxaoctadioic acid, 3, 5, 9-trioxaundecanedioic acid, 3,6,9,12-tetraoxatetradecanedioic acid and 3,6,9,12,15-pentaoxaheptadecanedioic acid,
said emollient can be selected from the group consisting of mineral oil, petrolatum, paraffin, ceresin, ozokerite, microcrystalline wax, polysiloxanes, silicone-glycol copolymers, triglyceride esters, acetylated monoglycerides, ethoxylated glycerides, alkyl esters of fatty acids, fatty acids and alcohols, lanolin and lanolin derivatives, polyhdric alcohol esters, sterols, beeswax derivatives, polyhydric alcohols and polyethers, and amides of fatty acids and
said emulsifier can be selected from the group consisting of sorbitans, alkoxylated fatty alcohols, alkylpolyglycosides, soaps, alkyl sulfates, monoalkyl and dialkyl phosphates, alkyl sulphonates, and acyl isothionates. Both the effective amount and the frequency of application will vary depending on the particular skin condition treated, the age and physical condition of the person under treatment, the severity of the condition, the duration of treatment, the nature of concurrent treatments, the specific compound or compositions employed, the particular vehicle utilized to deliver the compound or compositions, and other like factors within the knowledge and expertise of those skilled in the art.

The efficacy of the oxa compounds in treating skin conditions has been found to be affected by the pH of the composition. It is believed desirable to maintain the pH of the composition in the acid range pH < 7.0, preferably pH < 5.0, most preferably in the pH range between 3.5 and 4.0. The pH of the composition can be adjusted by adding water soluble salts formed by strong bases (e.g. KOH, NaOH, NHOH) and weak acids (e.g. phosphoric acid, acetic acid, lactic acid, carbonic acid). Examples of such salts include potassium biphosphate, sodium phosphate, sodium acetate, sodium lactate and the like. Other methods useful for adjusting the pH of topical compositions are known to those skilled in the art.

Compositions of the present invention have clear advantages over alpha hydroxyacid formulations, including superior mildness. Formulations containing alpha hydroxyacids, such as glycolic and lactic acids, can cause substantial discomfort to some individuals and symptoms of severe skin irritation in others, upon facial application. For instance, a formulation containing 4.0% glycolic acid at pH 3.7 produced a skin irritation (PII) score of 0.23 when tested on 20 panelists. In contrast, a composition containing 10% of 3,6,trioxaundecanedioic acid at pH 3.7 produced a PII score of only 0.13 (see Example 2, below).

Compositions that manifest a PII score of less than or equal to 0.15 are considered non-irritating; those exhibiting PII scores between 0.15 and 0.3 are considered moderately irritating; and compositions that elicit a PII score of more than 3.0 from tested panelists are considered serious irritants. The PII scoring takes into consideration such factors as the number of panelists displaying irritation symptoms compared to the total number of panelists in the test.

While being significantly gentler to skin than glycolic acid formulations, the oxa diacid compositions of the present invention are highly effective in normalizing the desquamation of the upper stratum corneum, an activity required for the alleviation of the skin conditions listed above.

The topical compositions of the present invention also have advantages over compositions containing dicarboxylic acids including better water solubility and superior stratum corneum desquamatory activity. Oxa diacids easily dissolve in water to concentrations of at least 20 to 30% by weight and, therefore, allow a much wider range of composition flexibility. Dicarboxylic acids of moderate to long chain length, which have straight, all-carbon backbones, are virtually insoluble in water and other aesthetically acceptable vehicle. This severely limits the choice of delivery vehicles for the dicarboxylic acids. The desquamatory activity of such dicarboxylic acids is also questionable. For example, formulations containing 5% and 10% dodecanedioic acid do not produce any normalizing effect on stratum corneum desquamation beyond that of its vehicle alone.

Some specific examples of vehicles found to be suitable for use with the oxa compounds include;
(1) (a) about 2 wt% to about 10 wt% glycerin, (b) about 1 wt% to about 10 wt% propylene glycol, (c) about 0.1 wt% to about 2 wt% hydroxyethyl cellulose, (d) about 0.1 wt% to about 1 wt% imidazolidilyl urea, and (e) about 0.01 wt% to about 2 wt% disodium-EDTA;
(2) (a) about 1 wt% to about 10 wt% glycerin, (b) about 1 wt% to about 10 wt% propylene glycol, (c) about 1 wt% to about 10 wt% octyl palmitate, (d) about I wt% to about 10 wt% myristyl myristate, (e) about 1 wt% to about 6 wt% cetearyl alcohol/ Ceteareth-20, (f) about 0.5 wt% to about 6 wt% glyceryl monostearate, (g) about 0.1 wt% to about 2 wt% hydroxyethyl cellulose, (h) about 0.1 wt% to about 1 wt% imidazolidilyl urea, about 0.05 wt% to about 0.5 wt% methyl paraben, and (J) about 0.01 wt% to about 2 wt% disodium-EDTA; and
(3)(a) about 2 wt% to about 10 wt% glycerin, (b) about 1 wt% to about 10 wt% octyl palmitate, (c) about 1 wt% to about 10 wt% myristyl myristate, (d) about 1 wt% to about 7 wt% cetearyl alcohol/ Ceteareth-20, (e) about 1 wt% to about 10 wt% propylene glycol, (f) about 1 wt% to about 6 wt% glyceryl monostearate, (g) about 0.1 wt% to about 2 wt% hydroxyethyl cellulose, (h) about 0.1 wt% to about 1 wt% imidazolidilyl urea, (i) about 0.05 wt% to about 0.5 wt% methyl paraben, and (j) about 0.01 wt% to about 2 wt% disodium-EDTA.

The topical compositions of the present invention can be made as lotions. A first or more basic lotion comprises about 0.1 to about 90%, preferably from about 1 to about 50%, and most preferably about 5 to about 20% of the oxa diacid, and the remainder water. A second lotion has about 0.1 to about 90%, preferably from about 1 to about 50%, and most preferably about 5 to about 20% of the oxa diacid, about 0.5 to about 50% of an emollient about 0.1 to about 30% of an emulsifier and the remainder water. The second lotion may also contain up to about 10% of a preservative, from about 0.1 to about 3% of a fragrance, and up to about 5% of a dye or a pigment.

The topical composition of the invention can also be formulated as a cream. A first or more basic cream comprises about 0.1 to about 95%, preferably from about 1 to about 50%, and most preferably about 5 to about 20%_of the oxa diacid, from about 0.5 to about 50% of an emollient, about 0.1 to about 6% of a thickener and the remainder water. A second, preferred cream comprises about 0.1 to about 90%, preferably from about 1 to about 50%, and most preferably about 5 to about 20% of the oxa diacid, from about 0.5 to about 50% of an emollient, about 0.1 to about 30% of an emulsifier, about 0.1 to about 6% of a thickener and the remainder water.

The oxa diacid can be combined with most conventional emollients including mineral oil, petrolatum, paraffin, ceresin, ozokerite, microcrystraline wax, perhydrosqualene dimethyl polysiloxanes, methylphenyl polysiloxanes, silicone-glycol copolymers, triglyceride esters, acetylated monoglycerides, ethoxylated glycerides, alkyl esters of fatty acids, fatty acids and alcohols, lanolin and lanolin derivatives, polyhydric alcohol esters, sterols, beeswax derivatives, polyhydric alcohols and polyethers, and amides of fatty acids. Other suitable emollients can be found in Sagarin, Cosmetics, Science and Technology, 2nd Ed., vol. 1, pp. 32-43 (1972).

The emulsifiers that can be cationic, anionic, nonionic or amphoteric, or a combination thereof. Nonionic emulsifiers are preferred. Exemplary nonionic emulsifiers are commercially available sorbitans, alkoxylated fatty alcohols and alkyl polyglycosides. Anionic emulsifiers may include soaps, alkyl sulfates, monoalkyl and dialkyl phosphates, alkyl sulphonates and acyl isothionates. Other suitable emulsifiers can be found in McCutcheon, Detergents and Emulsifiers, North American Edition, pp. 317-324 (1986).

The preservatives suitable for use with the present compositions include alkanols, especially ethanol and benzyl alcohol, parabens, sorbates, urea derivatves, and isothiazolinones.

While such lotions or creams can be made using conventional homogenization methods known to those skilled in the art, it is also possible to use a process of microfluidization that involves co-mixing the aqueous phase and the oil phase of such creams and lotions in a high-pressure homogenizer that reduces the emulsion particle size dramatically to about 1/400th the size of those in creams and lotions prepared without applying high pressure. Microfluidization allows one to prepare elegant stable creams and lotions containing effective amounts of an oxa diacid without the use of traditional emulsifiers and surfactants.

The topical compositions of the invention can also be formulated as a micro-emulsion. A first, basic micro-emulsion system comprises about 0.1 to about 50%, preferably from about 1 to about 30%, and most preferably about 5 to about 20% of the oxa diacid, from about 0.5 to about 20% of a hydrocarbon, from about 0.5 to about 20% of an oil, and the remainder water. A second, more preferred micro-emulsion system comprises about 1 to about 20% of the oxa diacid, from about 0.5 to about 15% of a hydrocarbon, from about 1 to about 15% of an oil, from about 0.1 to about 10% of a fatty alcohol, up to 30% of an nonionic surfactant, and the remainder water.

The topical compositions of the invention can be formulated as oil-in-water or water-in-oil emulsions, gels, lotions, ointments, sticks, sprays, tapes, patches. The inventive compositions can also be in the form of a multiphase emulsion, such as a water-in-oil-in-water type emulsion as disclosed in U.S. Patent No. 4, 254,105. The compositions of the invention can also be formulated as triple emulsions of the oil-in-water-silicone fluid type disclosed in U.S. Patent No. 4, 960, 764.

The compositions of the invention can also be made as a liposomal formulation, for example, according to the methods d scribed in Mezei, J. Pharmaceut. Pharmacol., vol. 34, pp. 473-474 (1982), or modification thereof. In such compositions, droplets of the oxa diacid solution can be entrapped inside the liposomal vesicles with the shell of the liposome being a phospholipid or other suitable lipids (e.g. skin lipids). To form a topical composition, the liposomes can then be added to any of the carrier systems described above according, for example, to the preparation modes, uses and compositions of topical liposomes described in Mezei, Topics in Pharmaceutical Sciences, Breimer et al. Eds., pp..345-358, Elsevier Science Publishers BV, New York (1985) or according to the reverse-phase evaporation method described in Szoka et al., Proc. Nat. Acad. Sciences, vol. 75, pp. 4194-4198 (1978), and Diploses et al., J. Soc. Cosmetic Chemists, vol. 43, pp93-100 (1992). Solutions of oxa diacids can also be entrapped in polymeric vesicles with a shell consisting of a suitable polymeric material such as gelatin, cross-linked gelatin, polyamide, poylacrylates and the like to form a vesicle that is then incorporated into the topical composition.

The compositions of the invention can include only an oxa compound as an active ingredient, or can use the oxa compound in combinations with other cosmetic and pharmaceutical actives and excipients. Suitable other cosmetic and pharmaceutical agents include, but are not limited to, antifungals, vitamins, sunscreens, retinoids, antiallergenic agents, depigmenting agents, anti-inflammatory agents, anesthetics, surfactants, moisturizers, exfolients, stabilizers, preservatives, antiseptics, thickeners lubricants, humectants, chelating agents and skin penetration enhancers, as well as the emulsifiers, emollients, fragrances and colorants discussed above.

Examples of suitable thickening agents include xanthan gum, xanthan gum brine tolerant, hydroxypropyl cellulose, hydroxyethyl cellulose, carbopol and gum acacia, Sepigel 305 (available from Seppic Co., France), vee-gum or magnesium aluminum silicate.

In topical compositions, oxa diacids are also compatible with, and their utility can be enhanced by, humectants such as urea, PCA, amino acids, certain polyols and other compounds with hygroscopic properties.

Topical compositions can also be formed to contain about about 0.1 to about 90%, preferably from about 1 to about 50%, and most preferably about 5 to about 20% of the oxa diacid, in combination with a keratolytic agent, such as salicylic acid and benzoyl peroxide, and skin lightening agents such as kojic acid benzoquinone, licorice derivatives, ascorbic acid and its derivatives (e.g. magnesium ascorbyl phosphate), and glycerhetinic acid and its derivatives.

From about 0.1 to about 90%, preferably from about 1 to about 50%, and most preferably about 5 to about 20% of the oxa diacid can be used to form a topical formulation in combination with organic and inorganic sunscreens such as titanium dioxide, zinc oxide, benzylidene camphor, anthranilates, butylmethoxydibenzoylmethane, naphtholsulphonates and cinnamic acid derivatives Of these, butylmethoxydibenzoylmethane and cinnamic acid derivatives are preferred.

Topical compositions of the invention can also contain about 0.1 to about 90%, preferably from about 1 to about 50%, and most preferably about 5 to about 20% of the oxa diacids co-formulated with (i) retinoids such as retinol, retinoic acid, retinyl palmitate, retinyl propionate, retinyl acetate, isotretinoin as well as synthetic retinoid mimics; (ii) hormonal compounds such as estriol, estradiol, estrone or conjugated estrogens; (iii) alpha-hydroxyacids or polyhydroxy alpha-hydroxy acid such as glycolic acid, lactic acid, tartaric acid, gulonic acid and other carboxylic acids and their monomeric, polymeric, cyclic or acyclic derivatives; (iv) alpha-keto acids such as pyruvic acid, 2-oxopropanoic acid, 2-oxobutanoic acid, 2-oxopentanoic acid, and the like.

From about 0.1 to about 90%, preferably from about 1 to about 50%, and most preferably about 5 to about 20% of the oxa diacids can also be utilized for additional benefits in topical formulations containing one or more of the following:
(i)vitamins including, for example, enzyme cofactors such as vitamin B6, vitamin B12, vitamin D3, 1,25-dihydroxy vitamin D3, vitamin B1, vitamin B2 , vitamin K, vitamins E, tocotrienols and their derivatives, nicotinic acid and its esters, pantothenic acid and it esters, panthenol, folic acid and its derivatives, choline, carnitine and substances without formal vitamin status or "pseudo vitamins" such as vitamin F or cis,cis-linoleic acid, vitamin M or pteroylglutamic acid, vitamins B10 and B11, sesame seed factor, termitin, penicin, insectine, hypomycin and mycoine, vitamin L or anthranilic acid, vitamin L2 or adenylthiomethyl-pentose, myoinositol or cis-1, 2, 3, 5-trans-4-6-cyclohexanehexol and its esters, especially phytic acid, laetrile or 1-mandelo-nitrile-beta-glucuronic acid, amygdalin, vitamin B15 or pangamic acid, vitamin B13 or orotic acid, vitamine H3 or procaine hydro-chloride, vitamin U or methyl-sulfonium salts of methionine, and pyrroloquinoline quinone;
(ii) antifungal agents including, for example, clotrimazole, ketoconazole, miconazole, naftifine, tolnaftate, amphotericin B, nystatin, 5-fluorocytosine, griseofulvin, haloprogin, of which tolnaftate, haloprogin and miconazole are most preferred;
(iii) self-tanning agents including, for example, as dihydroxyacetone and lawsone, of which dihydroxyacetone is most preferred;
(iv) anti-mycobacterial agents such as erythromycin, tetracyclin and related compounds, especially doxycyclin and methacyclin, cephalosporins, penicillins, macrolides, peptide compounds such as novobiocin, vancomycin, oleandomycin paromomycin, leucomycine, amphomycin with macrolide molecules, quinolone derivatives and other compounds that interfere with bacterial cell wall synthesis, membrane function, RNA metabolism, purine, pyrimidine and protein synthesis, respiration or phosphorylation;
(v) topical analgesics such as lidocaine, benzocaine, butacaine, tetracaine, clove oil and eugenol, of which benzocaine and lidocaine are most preferred;
(vi) lipidic compounds essential for the skin's barrier function including, for example, ceramides, essential fatty acids and their esters, especially glycerides, ω-hydroxy fatty acids and their esters derived with alkanols through carboxylic hydroxyl or with, other fatty acids at the omega-hydroxyl, the latter type being most preferred, with phospholipids, cholesterol and its esters, such as cholesteryl hemisuccinate and cholesteryl phospate of which cholesterol phospate and essential fatty acids are most preferred, phytosterols, cholestanol and its derivatives. The lipidic compounds can be added to a topical composition either as singular molecular entities or as a complex mixture of lipids derived from either synthetic, animal or plant sources;
(vii) antiallergenic agents and H1 and/or H2,antihistamines such as diphenylhydramine, clomisole, antazoline, thenaldine, phenyltoloxamine citrate, tricyclic antiallergenics such as ketotifene, dithiaden. and 3-thienylsulfide of thiadene, H2-receptor blockers, especially burimamide, metiamide and cimetidien, cromolic acid and its salts;
(viii) the oxa-diacids can be used with topical anti-inflammatory agents that can reduce inflammation. These anti-inflammatory agents are used at concentrations from about 0.025% to 10%, preferably, 0.5-1%, with the concentration of the anti-inflammatory adjusted upward or downward depending upon the potency of the utilized agents. Examples of steroidal anti-inflammatories that can be used with oxa diacids include hydrocortisone, hydroxytriamcilone, alphamethyl dexamethasone, dexamethasone phosphate, beclamethasone dipropionate, hydrocortisone valerate, hydrocortisone cyclopentylpropionate, prednisolone, and mixtures thereof, with the most preferred being prednisolone and hydrocortisone; and
(ix) non-steroidal anti-inflammatories can also be employed, such as described in Rainsford, Antiinflammatory and Anti-Rheumatic Drugs, Vols. I-III, CRC Press, Boca Raton, Florida (1985), and specific examples of suitable NSAID's including, for example, oxicams (e.g. piroxicam, isoxicam), fenamic acid derivatives, meclofenamic acid derivatives (e.g. sodium meclofenamate), flufenamic acid derivatives, mefenamic acid derivatives, propionic acid asters such as ibuprofen, naproxen, benoxaprofen, flubiprofen, ketoprofen, suprofen, of which ibuprofen is most preferred; pyrazolidinediones, of which phenylbutazone is most preferred; acetic acid derivatives, such as diclofenac, fenclofenac, indomethacin, sulindac, of which indomethacin is most preferred; salicylic acid derivatives, such as, for example, asprin, disalacid, and benorylate, of which aspirin and disalacid are most preferred.

The compositions of the invention may also include safe anti-inflammatory products of natural origin shown to possess anti-inflammatory activity such as aloe vera extracts, extracts from genus Rubis (Rubia Cordifolia), extracts from genus Commiphom (Commiphora Mukul), willow bark, matricarria flowers, arnica flower, comfrey root, fenugreek seed and the like known to those skilled in the art.

Topical compositions of the invention can contain from about 0.1 to about 90%, preferably from about 1 to about 50%, and most preferably about 5 to about 20% of the oxa diacids in combination with antioxidants with phenolic hydroxy functions such as gallic acid derivataives (e.g. propyl gallate), bio-flavonoids (e.g. quercetin, rutin, daidzein, genistein), ferrulic acid derivatives (e.g. ethyl ferrulate, sodium ferrulate), 6-hydroxy-2,5,7,tetra-, methylchroman-2-carboxylic acid. The compositions may also contain effective concentrations of water soluble antioxidants such as uric acid, reductic acid, tannic acid, rosmarinic acid and catechins.

Also of benefit is a coformulation of from about 0.1 to about 90%, preferably from about 1 to about 50%, and most preferably about 5 to about 20% of the oxa diacids with nitric oxide synthase inhibitors that reduce skin redness, vasodilation and inflammatory reactions, especially in response to electromagnetic and ionizing radiation or to the action of chemically or biochemically aggresive compounds. The nitric oxide synthase inhibitors can be added at concentrations from about 0.05% to 10%, most preferably from 1% to 3%, and selected from the group including guanidine derivatives, especially monoaminoguianidine and methylguanidine, L-arginine derivatives, especially N^{G}-nitro-L-arginine and its esters, N^{G}-monomethyl-L-arginine, 2-iminopipperidines and other 2-iminoazaheterocycles.

Other possible antioxidants that the composition may contain are those that have one or more thiol functions (-SH), in either reduced or non-reduced form, such as glutathione, lipoic acid, thioglycolic acid, and other sulfhydryl compounds. The levels of sulfhydryl antioxidants should not exceed 0.5% for cosmetic uses of the composition but may be higher for pharmaceutical uses as dictated by the considerations of efficacy. The composition may also include inorganic antioxidants such as sulfites, bisulfites, metabisulfite, or other inorganic salts and acids containing sulfur in oxidation state +4. The preferred level of inorganic sulfur-containing antioxidants is from about 0.01% to about 0.5% with the most preferred level between about 0.1% and about 0.4% by weight.

Compositions of the invention can also include from about 0.1 to about 90%, preferably from about 1 to about 50%, and most preferably about 5 to about 20% of the oxa diacids coformulated with about 0.025% to 5%, with 0.5%-2% preferred and with 0.5-1% most preferred, of compounds known to be electron spin-traps such as nitrones, N-tertbutyl-nitrone and α-(4-pyridyl-1-oxide)-N-tertbutylnitrone or other compounds known to form free radicals with half-life times of more than one minute.

From about 0.1 to about 90%, preferably from about 1 to about 50%, and most preferably about 5 to about 20% of the oxa diacids can also be used in compositions that contain insect repellents such as aliphatic, cyclic or aromatic amides, citronella oil, terpineol, cieole, neem oil and terephthalic acid and its esters. Other suitable insect repellents can be found in Technical Bulletin No. 1549 from the U.S. Department of Agriculture or in their Agricultural Handbook Nos. 69, 340 and 461.

The oxa diacid-containing topical compositions of the invention can also contain skin cooling compounds such as, by way of example, menthol, menthyl glycerol, assymetical carbonates, thiocarbonates and urethanes, N-substituted carboxamides, ureas or phosphine oxides such as described in J. Cosmet. Chem., vol. 29, p. 185 (1978), menthyl lactate, and menthone glycerine acetal.

The general activity and mildness to skin of the present topical compositions can also be enhanced by neutralization to pH 3.5 to 7.0, most preferably from pH 3.7 to 5.6, with one or more amphoteric and pseudoamphoteric compounds such as glycine, alanine, valine, serine, thionine, methionine, leucine, asparagine, histidine, glutamic acid, glutamine, lysine, cystine, cystein, tryptophan, serine, phenylalanine, citrulline, creatine, proline, 3- or 4-hydroxyproline, 5-hydroxylysine, ornithine and its derivatives, 3-aminopropanoic acid and other aminocarboxylic acids, canavanine, canaline, homoarginine, taurine, aminoaldonic acids and aminosugars, aminouronic acid, aminoaldaric acid, deacetylated hyaluronic acid, hyalobiuronic acid, chondrosine, desulfated heparin, neuraminic or sialic acid, methionine sulfone, glycylglycine, chondroitin, D,L-sphingosine, sphingomyelin, ophidine, glucagon, homocarnosine, phosphatidyl serine, cocoamphoglycine, phosphatidyl ethanolamine, cysteinesulfinic acid, glutathione, amphoteric inorganic oxides, polyamidoamines, polyamidoamine-based dendrimers, sodium hydroxymethylglycinate and polyethylene amine.

The utility and mildness of the present topical compositions can also be enhanced by certain chelating agents incorporated into the composition at levels from about 0.01% to about 25% by weight, more preferably from about 0.5% to 10%, and most preferably from about 1% to about 5%. Suitable examples of chelating agents include those that have a high affinity for zinc, calcium, magnesium, iron and/or copper ions, such as ethylene-diamine-tetra-acetic acid, (ethylenedioxy)-diethylene-dinitrilo-tetra-acetic acid, salicylaldoxime, quinolinol, diaminocyclohexane-tetra-acetic acid, diethylene-triaminopenta-acetic acid, dimethylglyoxime, benzoin oxime, triethylenetetramine, desferrioxamine or mixtures thereof.

The present invention also includes methods by which these compounds can be used to address the aforementioned skin conditions. Such methods include topically applying an effective amount of one or more compound of Formula (I) to the affected skin areas, normally once or twice daily. Such methods also include topically applying a composition containing an effective amount of one or more compounds of Formula (I) in a physiologically acceptable vehicle to the affected skin areas, normally once or twice daily. The methods of the present invention include the topical application of the compounds of Formula (I) in concentrations of up to 100%, when such compounds are a liquid at ambient temperature (e.g. 3,6,9-trioxaun-decanedioic acid), and when using the oxa compounds, for example, for skin peels or for softening hair.

The following examples are illustrative of the present invention.

### EXAMPLES

The compositions of the present invention are generally made into lotions, creams or gels for topical application.

### EXAMPLE 1

### Preparation of Oxa diacid Topical compositions

In a suitable vessel, water, glycerin, propylene glycol Na₂EDTA and trioxaundecanoic acid are added and mixed together. Ammonium hydroxide is added to the vessel in increments to adjus pH to the desired range. This pH-adjusted phase is then heated to 170-175°F. Hydroxyethyl cellulose is next added with agitation until uniform to complete phase A.

For the lotion and cream, phase B is added to a suitable, second vessel, combined and heated to 170-175°F. Phase B is then added to phase A with sufficient mixing, again at 170-175°F. The batch is then cooled to 120°F. Phase C is added to the batch and mixed until uniform.

| Phase | | GEL | LOTION | CREAM |
|---|---|---|---|---|
| (A) | water | Q.S. | Q.S. | Q.S. |
| | glycerin | 5.00 | 3.00 | 5.00 |
| | propylene glycol | 3.00 | 3.00 | 3.00 |
| | disodium-EDTA | 0.10 | 0.10 | 0.10 |
| | 3, 6, 9-trioxa-undecanoic acid | 10.00 | 10.00 | 10.00 |
| | hydroxyethyl cellulose | 0.50 | 0.30 | 0.500 |
| | ammonium hydroxide (30%) | to pH 3.7-3.9 | to pH 3.7-3.9 | to pH 3.7-3.9 |
| (B) | octyl palmitate | - | 3.00 | 5.00 |
| | myristyl myristate | - | 3.00 | 5.00 |
| | glyceryl monostearate | - | 1.50 | 3.00 |
| | cetearyl alcohol & Ceteareth-20 | - | 3.00 | 5.00 |
| | methyl paraben | - | 0.20 | 0.20 |
| (C) | imidazolidilyl urea | 0.30 | 0.30 | 0.30 |
| All numbers are expressed as percentages of total weight of composition except for pH ranges and Q.S. for balance with water. | | | | |

Those skilled in the art will readily perceive possible vehicles other than lotions, creams or gels, after having the benefit of this disclosure.

Microscopic normalization of desquamation of the stratum corneum or macroscopic exfoliation of the epidermis are prerequisite activities for alleviating the skin conditions for which the present oxa diacid compounds and compositions are intended. The following example demonstrates, inter alia, the superior stratum corneum desquamatory activity provided by the present oxa diacid compositions.

### EXAMPLE 2

### Exfoliation Patch Test for Desguamatory Activity

In general, the exfoliation patch test procedure involves a 24-hour occlusive patching to a skin site. Skin gradings are conducted immediately, and 24 hours after, removal of the patch. The test focuses primarily on product effects on the stratum corneum and mainly on exfoliation.

A corneocyte removing activity sampling (a "CRAS") is taken following the visual grading at 24 hours after removal of the patch. A CRAS score is a quantitative measure of corneocyte desquamation and its calculation is based on the amount of corneocytes removed with each sampling.

A series of studies were conducted, the first of which showed that in a 1-day exfoliation CRAS assay, a 10 wt% 3,6,9-trioxaundecanedioic acid composition at pH 3.7 had superior exfoliating activity compared to a formulation containing 4 wt% glycolic acid at pH 3.8. This study examined the exfoliation properties of 10 wt% oxa diacid at low and high pH, and 5 wt% oxa diacid at low pH. The 4 wt% glycolic acid was included as a frame of reference. Table 1, below, provides a complete data summary as well as material identification.

**Table 1**

| | pH | CRAS |
|---|---|---|
| 5 wt-% oxa diacid | 3.7 | 2.58 |
| 10 wt% oxa diacid | 3.7 | 2.93 |
| 10 Wt% oxa diacid | 5.4 | 2.68 |
| 4 wt% glycolic acid | 3.8 | 2.80 |

No significant irritation was observed with any sanple. In all of the various comparisons, the 10% oxa diacid low pH sample was consistently better and exhibited meaningful exfoliation activity.

The next study in the series re-confirmed that a composition with 10 wt% of 3,6,9-trioxaundecanedioic acid has an exfoliating activity superior to that of a 4 wt% glycolic acid formulation. At the same time, the 3,6,9-trioxaundecanedioic acid composition was also milder to the skin than the glycolic acid. It is to be noted that, on a molar basis, the concentration of "acid" in a 10% 3,6,9-trioxaundecanedioic acid composition is lower than that in a 4% glycolic acid formula. Coupled with the clinical results presented herein, this indicates that the intrinsic exfoliating activity of 3,6,9-trioxaundecanedioic acid is significantly higher than that of glycolic acid.

The second study was performed to confirm the results observed with the 10 wt% oxa diacid compositions at pH 3.7. Table 2, below, provides a data summary and material identification for the second study.

**Table 2**

| | pH | PII | CRAS | Previous CRAS |
|---|---|---|---|---|
| 10% oxa diacid | 3.7 | 0.13 | 3.23 | 2.93 |
| 10% oxa diacid | 3.74 | 0.18 | 3.33 | ---- |
| 4% glycolic acid | 3.7 | 0.23 | 3.05 | 2.80 |

No significant irritation was observed with any of the sampled participants, but use of the oxa diacids did provide lower irritation index (PII) scores in comparison to both the glycolic acid and vehicle. The results confirmed what was observed in the exfoliation assay of Example 2.

### EXAMPLE 3

### Cream for Hyperpigmented Spots

This example illustrates a cream that can be prepared and used to reduce appearance of hyper-pigmentation spots on the skin of hands.

| | W/W% |
|---|---|
| isopropyl myristate | 3.0 |
| polyethylene glycol (1000) monostearate | 5.0 |
| palmitic acid | 10.0 |
| 3,6,9,12-tetraoxatetradecanedioic acid | 10.0 |
| glycerine. | 3.0 |
| polyethylene glycol (300) monostearate | 5.0 |
| methyl paraben | 0.2 |
| magnesium ascorbyl phosphate | 2.0 |
| water | 60.0 |
| perfume & color | to 100.0 |
| | |
| triethanolamine | to pH 4.0 |
| All numbers are expressed as percentages of total weight of compositions except for reference to pH. | |

### EXAMPLE 4

### Cream for Dry Skin, Ichthvosis and Hyperkeratoses

This example illustrates a silicone cream that can be prepared and used to treat dry skin, ichthyosis and hyper-keratoses according to the present invention.

| | W/W% |
|---|---|
| Phase A | |
| laurylmethicone copolyol | 2.0 |
| mineral oil | 1.0 |
| lanolin | 1.5 |
| sunflower or soybean oil | 10.0 |
| cyclomethicone | 6.0 |
| oil soluble rosmary extract | 2.0 |

| Phase B | |
|---|---|
| sodium iodide | 2.0 |
| 3,6,9-trioxaundecandioic acid | 9.0 |
| 3,6,9,12,15-pentaoxaheptadecanedioic acid | 1.0 |
| sodium hydroxymethyl glycinate | 0.5 |
| demineralized water | to 100.0 |
| | |
| sodium hydroxymethyl glycinate | to pH 3.8 |
| All numbers are expressed as percentages of total weight of composition except for the reference to pH. | |

### EXAMPLE 5

### Silicone Gel

This example illustrates a water-in-silicone gel composition.

| | W/W% |
|---|---|
| Phase A | |
| dimethiconol | 10.0 |
| dimethicone copolyol | 10.0 |
| cyclomethicone | 5.0 |
| | |

| Phase B | |
|---|---|
| 3,6,9-trioxaundecanedioic acid | 8.0 |
| glycerine | 20.0 |
| demineralized water | to 100.0 |
| | |
| triethanolamine | to pH 4.0 |
| All numbers are expressed as percentages of total weight of composition except for the reference to pH. | |

### EXAMPLE 6

### Cream for Acne, Skin Blemishes and Age Spots

This example illustrates a face cream than can be used to treat acne, skin blemishes and age spots.

| | W/W% |
|---|---|
| Phase A | |
| oleic acid | 1.0 |
| stearic acid | 17.0 |
| polyoxyethylene (20 propylene glycol | |
| monostrearate) | 10.0 |
| retinol | 0.1 |

| Phase B | |
|---|---|
| glycerine | 5.0 |
| 2-pyrollidone-5-carboxylic acid | 5.0 |
| 3,6,9-trioxaundecanedioic acid | 7.5 |
| 3,6,9,12-tetraoxatetradecanedioic acid | 2.5 |
| lactic acid | 3.0 |
| demineralized water | to 100.0 |
| | |
| ammonium hydroxide | to pH 4.2 |
| All numbers are expressed as percentages of-total composition except for the reference to pH. | |

Various modifications and alterations to the present invention may be appreciated based on a review of this disclosure.

For the invention the following modifications are preferred :
A topical vehicle comprising an ingredient selected from the group consisting of ammonium hydroxide, cetearyl alcohol/Ceteareth-20, EDTA, glycerin, glyceryl monostearate, hydroxyethyl cellulose, imidazolidilyl urea, methyl paraben, myristyl myristate, octyl palmitate and propylene glycol and mixtures thereof;
Or a topical vehicle comprising about 0.1 wt% to about 20 wt% 3,6,9-trioxaundecanoic acid and a vehicle;
Or a topical vehicle comprising
   (a) about 2 wt% to about 10 wt% glycerin;
   (b) about 1 wt% to about 10 wt% propylene glycol;
   (c) about 0.1 wt% to about 2 wt% hydroxyethyl cellulose;
   (d) about 0.1 wt% to about 1 wt% imidazolidilyl urea;
   (e) about 0.01 wt% to about 2 wt% disodium-EDTA; and
   said composition has a pH of about 7.0 or less.
Or a topical vehicle comprising
   (a) about 1 wt% to about 10 wt% glycerin;
   (b) about 1 wt% to about 10 wt% propylene glycol;
   (c) about 1 wt% to about 10 wt% octyl palmitate;
   (d) about I wt% to about 10 wt% myristyl myristate;.
   (e) about 1 wt% to about 6 wt% catearyl alcohol/Ceteareth-20;
   (f) about 0.5 wt% to about 6 wt% glyceryl monostearate;
   (g) about 0.1 wt% to about 2 wt% hydroxyethyl cellulose;
   (h) about 0.1 wt% to about 1 wt% imidazolidilyl urea; about 0.05 wt% to about 0.5 wt% methyl paraben;
   (J) about 0.01 wt% to about 2 wt%. disodium-EDTA; and
   said composition has a pH of about 7.0 or less.
Or a topical vehicle comprising
   (a) about 2 wt% to about 10 wt% glycerin;
   (b) about 1 wt% to about 10 wt% octyl palmitate;
   (c) about 1 wt% to about 10 wt% myristyl myristate;
   (d) about 1 wt% to about 7 wt% cetearyl alcohol/Ceteareth-20;
   (e) about 1 wt% to about 10 wt% propylene glycol;
   (f) about 1 wt% to about 6 wt% glyceryl monostearate;
   (g) about 0.1 wt% to about 2 wt% hydroxyethyl cellulose;
   (h) about 0.1 wt% to about 1 wt% imidazolidilyl urea;
   (i) about 0.05 wt% to about 0.5 wt% methyl paraben;
   (j) about 0.01 wt% to about 2 wt% disodium-EDTA; and
   said composition has a pH of about 7.0 or less.

## Claims

1. A topical composition comprising a suitable topical vehicle and a compound of Formula (I): 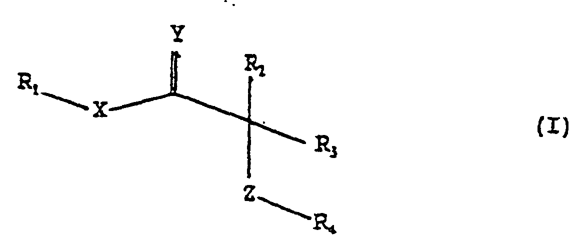 wherein R₄ is (CR₅R₆-CR₇R₈-X₁) ₙ-CR₉R₁₀-C (=X₂) X₃R₁₁, n is an integer from 1 to 18; R₁, R₂, R₃, R₅, R₆, R₇, R₈, R₉, R₁₀ and R₁₁, are independently, hydrogen or non-hydrogen substituents; and X, X₁, X₂, X₃, Y and Z are independently, 0, NH, or S, and wherein the non-hydrogen substituents are selected from the group consisting of alkyls , alkenyls, oxaalkyls and aryls.

2. The composition of claim 1, wherein said composition comprises a compound of Formula (I) with about 0.1 to about 95 wt% of said composition.

3. The composition of claim 2, wherein said composition comprises a compound of formula (I) with about 1 to about 50 wt% of said composition.

4. The composition of claim 3, wherein said composition comprises a compound of formula (I) with about 5 to about 20 wt% of said composition.

5. The composition of claim 1, wherein n is an integer from 2 to 12.

6. The composition of claim 1, further comprising a mixture of at least two different compounds of Formula (I).

7. The composition of claim 1, wherein said non-hydrogen substituents are selected from the group consisting of methyl, ethyl, propyl, isopropyl, butyl, isobutyl, hexyl, heptyl, octyl, nonyl, dodecanyl, me'thoxy, ethoxy, propoxy, butoxy, cyclohexenyl, hydroxymethyl, hydroxyethyl, hydroxypropyl, cyclobutyl and cyclohexanyl.

8. The composition of claim 1, wherein R₁, R₂, R₃, R₅, R₆, R₇, R₈, R₉, R₁₀ and R₁₁, are each hydrogen.

9. The composition of claim 1, wherein X, X₁, X₂, X₃, Y and Z are each oxygen.

10. The composition of claim 1, wherein X, X₁, X₂, X₃, Y and Z are each amino groups.

11. The composition of claim 1, wherein X, X₁, X₂, X₃, Y and Z are each sulfur.

12. The composition of claim 1, wherein said compound is selected from the group consisting of 3,6-dioxaoctadioic acid; 3,6,9-trioxaundecanedioic acid; 3,6,9,12- tetraoxatetradecanedioic acid; 3,6,9,12,15-pentaoxaheptadecanedioic acid; 2-methyl-3,6,9-trioxaundecanedioic acid; 2-ethyl-3,6,9,12-tetraoxatetradecanedioic acid; 2-phenyl-3,6,9-trioxaundecanedioic acid; 3,6,9-trioxaundecanedioic acid diethyl ester; 3,6,9-triaminoundecanedioic acid; 3,6,9,12-tetraminotetradecanedioic acid; 3-amino-6,9-dioxa-undecanedioic acid; 3,6-diamino-9-xaundecanedioic acid; 3,6,9-trithioundecahedioic acid; 3,6-dithio-9,12-dioxatetradecanedioc acid; 3-amino-6,9-dioxaundecanedioic acid monoamide; 3-amino-6,9-dioxaundecanedioic acid diamide; 3,6,9-trioxaundecanedioic acid monoamide; 3,6,9-trioxaundecanedioic acid diamide; 2-10-dimethyl-3,6,9-trioxaundecanedioic acid; 2,10 -dimethyl-3,9-dithio-6-oxaundecanedioic acid; and mixtures thereof.

13. The composition of claim 1, wherein said vehicle is selected from the group consisting of lotion, cream and gel.

14. The composition of claim 1, further comprising at least one active selected from the group consisting of antifungals, vitamins, sunscreens, retinoids, antiallergenic agents, depigmenting agents, anti-inflammatory agents, anesthetics, surfactants, moisturizers, exfolients, emulsifiers, stabilizers, preservatives, antiseptics, emollients, thickeners, lubricants, humectants, chelating agents, fragrances, colorants and skin penetration enhancers.

15. The composition of claim 1, wherein said composition has a pH of less than 7.0.

16. The composition of claim15, wherein said pH is about 3.5 to about 7.0

17. The composition of claim16, wherein said pH is about 3.5 to about 4.0.

18. The composition of claim 2, wherein said composition comprises about 1 to about 50 wt% of said compound of Formula (I) and has a pH of less than 7.0.

19. The composition of claim15, wherein said topical vehicle comprises 0.1 to about 95 wt% of said compound of formula (I) ; 0.5 to about 50 wt% of an emollient; and about 0.1 to about 30 wt% of an emulsifier.

20. The composition of claim 19, wherein said compound is selected from the group consisting of 3,6-dioxaoctadioic acid, 3,6,9-trioxaundecanedioic acid, 3,6,9,12-tetraoxatetradecanedioic acid and 3,6,9,12,15-pentaoxaheptadecanedioic acid; and wherein
said emollient is selected from the group consisting of mineral oil, petrolatum, paraffin, ceresin, ozokerite, microcrystalline wax, perhydrosqualene, dimethyl polysiloxanes, methylphenyl polysiloxanes, silicone-glycol copolymers, triglyceride esters, acetylated monoglycerides, ethoxylated glycerides, alkyl esters of fatty acids, fatty acids and alcohols, lanolin and lanolin derivatives, polyhdric alcohol esters, sterols, beeswax derivatives, polyhydric alcohols and polyethers, and amides of fatty acids; and wherein
said emulsifier is selected from the group consisting of sorbitans, alkoxylated fatty alcohols, alkylpolyglycosides, soaps, alkyl sulfates, monoalkyl and dialkyl phosphates, alkyl sulphonates, and acyl isothionates.

21. The composition of claim 2, wherein said composition is in a form selected from the group consisting of an oil-in-water emulsion, a water-in-oil emulsion, a water-in-oil-in-water emulsion and an oil-in-water-in silicone fluid emulsion.

22. The composition of claim 13, wherein said topical vehicle comprises an ingredient selected from the group consisting of ammonium hydroxide, cetearyl alcohol/Ceteareth-20, EDTA, glycerin, glyceryl monostearate, hydroxyethyl cellulose, imidazolidilyl urea, methyl paraben, myristyl myristate, octyl palmitate and propylene glycol and mixtures thereof.

23. The composition of claim 13, comprising about 0.1 wt% to about 20 wt% 3,6,9-trioxaundecanoic acid and a vehicle.

24. The composition of claim 21, wherein said vehicle comprises:
(a) about 2 wt% to about 10 wt% glycerin;
(b) about 1 wt% to about 10 wt% propylene glycol;
(c) about 0.1 wt% to about 2 wt% hydroxyethyl cellulose;
(d) about 0.1 wt% to about 1 wt% imidazolidilyl urea;
(e) about 0.01 wt% to about 2 wt% disodium-EDTA; and said composition has a pH of about 7.0 or less.

25. The composition of claim 23, wherein said vehicle comprises:
(a) about 1 wt% to about 10 wt% glycerin;
(b) about 1 wt% to about 10. wt% propylene glycol;
(c) about 1 wt% to about 10 wt% octyl palmitate;
(d) about I wt% to about 10 wt% myristyl myristate;
(e) about 1 wt% to about 6 wt% cetearyl alcohol/Ceteareth-20;
(f) about 0.5 wt% to about 6 wt% glyceryl monostearate;
(g) about 0.1 wt% to about 2 wt% hydroxyethyl cellulose;
(h) about 0.1 wt% to about 1 wt% imidazolidilyl urea; about 0.05 wt% to about 0.5 wt% methyl paraben;
(J) about 0.01 wt% to about 2 wt% disodium-EDTA; and said composition has a pH of about 7.0 or less.

26. The composition of claim 23, wherein said vehicle comprises:
(a) about 2 wt% to about 10 wt% glycerin;
(b) about 1 wt% to about 10 wt% octyl palmitate;
(c) about 1 wt% to about 10 wt% myristyl myristate;
(d) about 1 wt% to about 7 wt% cetearyl alcohol/Ceteareth-20;
(e) about 1 wt% to about 10 wt% propylene glycol;
(f) about 1 wt% to about 6 wt% glyceryl monostearate;
(g) about 0.1 wt% to about 2 wt% hydroxyethyl cellulose;
(h) about 0.1 wt% to about 1 wt% imidazolidilyl urea;
(i) about 0.05 wt% to about 0.5 wt% methyl paraben;
(j) about 0.01 wt% to about 2 wt% disodium-EDTA; and said composition has a pH of about 7.0 or less.

27. Use of an effective amount of a compound of Formula (I): 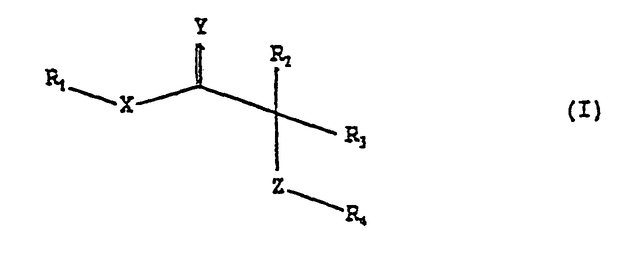 wherein R₄ is (CR₅R₆-CR₇R₈-X₁)ₙ-CR₉R₁₀-C(=X₂)X₃R₁₁, n is an integer from 1 to 18; R₁, R₂, R₃, R₅, R₆, R₇, R₈, R₉, R₁₀ and R₁₁, are independently, hydrogen or non-hydrogen substituents; and X, X₁, X₂, X₃, Y and Z are independently, 0, NH, or S, and wherein the non-hydrogen substituents are selected from the group consisting of alkyls, alkenyls, oxaalkyls and aryls for the manufacture of the medicament for treating skin conditions caused by accompanied with or exacerbated by abnormal desquanation.

28. Use acording to claim 27, wherein said compound is combined with a suitable topical vehicle in a topical composition.

29. use according to claim 27, further comprising, applying to said skin an effective amount of a mixture of two or more different compounds of Formula (I) .

30. Use according to claim 28, wherein said composition comprises about 0.1 to about 95 wt% of said compound of Formula (1)

31. Use according to claim 30, wherein said composition comprises about 1 to about 50 wt% of said compound of Formula (1).

32. Use according to claim 30, wherein n is an integer from 2 to 12.

33. Use according to claim, 32, wherein said non-hydrogen substituents of said compound are selected from the group consisting of methyl, ethyl, propyl, isopropyl, butyl, isobutyl, hexyl, heptyl, octyl, nonyl, dodecanyl, methoxy, ethoxy, propaxyl, butoxy, cyclohexenyl, hydroxymethyl, hydroxyethyl, hydroxypropyl, cyclobutyl and cyclohexanyl.

34. Use according to claim 27, wherein R₁, R₂, R₃, R₅, R₆, R₇, R₈, R₉, R₁₀, and R₁₁ are each hydrogen.

35. Use according to claim27,wherein X, X₁, X₂, X₃, Y and Z are each oxygen.

36. Use according to claim 27, wherein X, X₁, X₂, X₃, Y and Z are each amino groups.

37. Use according to claim 27, wherein X, X₁, X₂, X₃, Y and Z are each sulfur.

38. Use according to claim 27, wherein said compound is selected from the group consisting of 3,6-dioxaoctadioic acid; 3,6,9-trioxaundecanedioic acid; 3,6,9,12-tetraoxatetradecanedioic acid; 3,6,9,12,15-pentaoxaheptadecanedioic acid; 2-methyl-3,6,9-trioxaundecanedioic acid; 2-ethyl-3,6,9,12-tetraoxatetradecanedioic acid; 2-phenyl-3,6,9-trioxaundecanedioic acid; 3,6,9-trioxaundecanedioic acid diethyl ester; 3,6,9-triaminoundecanedioic acid; 3,6,9,12-tetraminotetradecanedioic acid; 3-amino-6,9-dioxa-undecanedioic acid; 3,6-diamino-9-oxaundecanedioic acid; 3,6,9-trithioundecanedioic acid; 3,6-dithio-9,12-dioxatetradecanedioic acid; 3-amino-6,9-dioxaundecanedioic acid monoamide; 3-amino-6,9-dioxaundecanedioic acid diamide; 3,6,9-trioxaundecanedioic acid monoamide; 3,6,9-trioxaundecanedioic acid diamide; 2-10-dimethyl-3,6,9-trioxaundecanedioic acid; 2,10-dimethyl-3,9-dithio-6-oxaundecanedioic acid; and mixtures thereof.

39. Use according to claim 38, wherein said compound is 3,6,9-trioxaundecanoic acid.

40. Use according to claim 28, wherein said composition further comprises at least one active - selected from the group consisting of antifungals, vitamins, sunscreens, retinoids, antiallergenic agents, depigmenting agents, anti-inflammatory agents, anesthetics, surfactants, moisturizers, exfolients, emulsifiers, stabilizers, preservatives, antiseptics, emollients, thickeners, lubricants, humectants, chelating agents, fragrances, colorants and skin penetration enhancers.

41. Use according to claim 27, wherein said skin conditions are selected from the group consisting of dry skin, ichthyosis, palmar and plantar hyperkeratoses, dandruff, lichen simplex chronicus, Dariers disease, keratoses, lentigines, age spots, melasmas, blemished skin, acne, psoriasis, eczema, pruritis, inflammatory dermatoses, striae distensae, warts, calluses, signs of dermatological aging, skin wrinkles, fine wrinkles around the mouth area, irregular pigmentation, sallowness, loss of skin resilience and elasticity, and disorders associated with nails, cuticles and hair.

42. A cosmetic method of peeling skin comprising, applying to said skin, a compound of formula I: 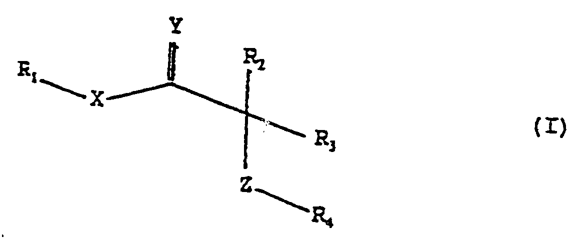 wherein R₄ is (CR₅-C₆CR₇R₈-X₁)ₙ-CR₉R₁₀-C(=X₂)X₃R₁₁, n is an integer from 1 to 18; R₁, R₂, R₃, R₅, R₆, R₇, R₈, R₉, R₁₀ and R₁₁, are independently, hydrogen or non-hydrogen substituents; and X, X₁, X₂, X₃, Y and Z are independently, 0, NH, or S, and wherein the non-hydrogen substituents are selected from the group consisting of alkyls, alkanyls, oxaalkyls and aryls.

43. A method of softening hair comprising, applying to said hair, a compound of Formula I: 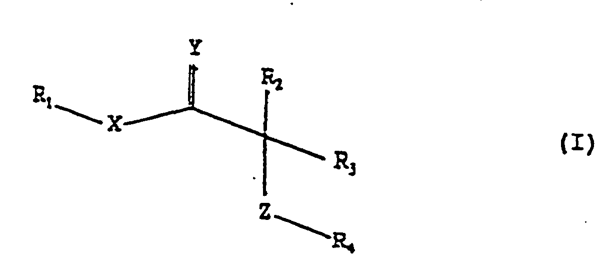 wherein R₄ is (CR₅R₆-CR₇R₈-X₁)ₙ-CR₉R₁₀-C(=X₂) X₃R₁₁, n is an integer from 1 to 18; R₁, R₂, R₃, R₅, R₆, R₇, R₈, R₉, R₁₀ and R₁₁, are independently, hydrogen or non-hydrogen substituents; and X, X₁, X₂, X₃, Y and Z are independently, 0, NH, or S, and wherein the non-hydrogen substituents are selected from the group consisting of alkyls, alkenyls, oxaalkyls and aryls.

## Patentansprüche

1. Topische Zusammensetzung, umfassend ein geeignetes topisches Vehikel und eine Verbindung der Formel (I): 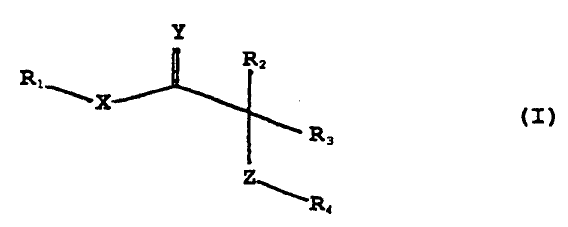 wobei R₄ (CR₅R₆-CR₇R₈-X₁)ₙ-CR₉R₁₀-C(=X₂)X₃R₁₁ ist, n eine ganze Zahl von 1 bis 18 ist; R₁, R₂, R₃, R₅, R₆, R₇, R₈, R₉, R₁₀ und R₁₁ unabhängig voneinander Wasserstoff oder Nicht-Wasserstoff-Substituenten sind; und X, X₁, X₂, X₃, Y und Z unabhängig voneinander O, NH oder S sind, und wobei die Nicht Wasserstoff Substituenten ausgewählt sind aus der Gruppe bestehend aus Alkylen, Alkenylen, Oxaalkylen und Arylen.

2. Zusammensetzung nach Anspruch 1, wobei die Zusammensetzung eine Verbindung der Formel (I) mit etwa 0,1 bis etwa 95 Gew.% der Zusammensetzung umfasst.

3. Zusammensetzung nach Anspruch 2, wobei die Zusammensetzung eine Verbindung der Formel (I) mit etwa 1 bis etwa 50 Gew.% der Zusammensetzung umfasst.

4. Zusammensetzung nach Anspruch 3, wobei die Zusammensetzung eine Verbindung der Formel (I) mit etwa 5 bis etwa 20 Gew.% der Zusammensetzung umfasst.

5. Zusammensetzung nach Anspruch 1, wobei n eine ganze Zahl von 2 bis 12 ist.

6. Zusammensetzung nach Anspruch 1, zudem umfassend ein Gemisch aus mindestens zwei verschiedenen Verbindungen der Formel (I).

7. Zusammensetzung nach Anspruch 1, wobei die Nicht-Wasserstoff-Substituenten ausgewählt sind aus der Gruppe, bestehend aus Methyl, Ethyl, Propyl, Isopropyl, Butyl, Isobutyl, Hexyl, Heptyl, Octyl, Nonyl, Dodecanyl, Methoxy, Ethoxy, Propoxy, Butoxy, Cyclohexenyl, Hydroxymethyl, Hydroxyethyl, Hydroxypropyl, Cyclobutyl und Cyclohexanyl.

8. Zusammensetzung nach Anspruch 1, wobei R₁, R₂, R₃, R₅, R₆, R₇, R₈, R₉, R₁₀, und R₁₁, jeweils Wasserstoff sind.

9. Zusammensetzung nach Anspruch 1, wobei X, X₁, X₂, X₃, Y und Z jeweils Sauerstoff sind.

10. Zusammensetzung nach Anspruch 1, wobei X, X₁, X₂, X₃, Y und Z jeweils Aminogruppen sind.

11. Zusammensetzung nach Anspruch 1, wobeil X, X₁, X₂, X₃, Y und Z jeweils Schwefel sind.

12. Zusammensetzung nach Anspruch 1, wobei die Verbindung ausgewählt ist aus der Gruppe, bestehend aus 3,6-Dioxaoctadisäure; 3,6,9-Trioxaundecandisäure; 3,6,9,12-Tetraoxatetradecandisäure; 3,6,9,12,15-Pentaoxaheptadecandisäure; 2-Methyl-3,6,9-trioxaundecandisäure; 2-Ethyl-3,6,9,12-tetraoxatetradecandisäure; 2-Phenyl-3,6,9-trioxaundecandisäure 3,6,9-Trioxaundecandisäurediethylester; 3,6,9-Triaminoundecandisäure; 3,6,9,12-Tetraminotetradecandisäure; 3-Amino-6,9-dioxaundecandisäure; 3,6-Diamino-9-oxaundecandisäure; 3,6,9-Trithioundecandisäure; 3,6-Dithio-9,12-dioxatetradecandisäure; 3-Amino-6,9-dioxaundecandisäuremonoamid; 3-Amino-6,9-dioxaundecandisäurediamid; 3,6,9-Triouaundecandisäuremonoamid; 3,6,9-Trioxaundecandisäurediamid; 2,10-Dimethyl-3,6,9-trioxaundecandisäure; 2,10-Dimethyl-3,9-dithio-6-oxaundecandisäure; und deren Gemische.

13. Zusammensetzung nach Anspruch 1, wobei das Vehikel ausgewählt ist aus der Gruppe, bestehend aus Lotion, Creme und Gel.

14. Zusammensetzung nach Anspruch 1, zudem umfassend mindestens einen Wirkstoff, ausgewählt aus der Gruppe, bestehend aus Fungiziden, Vitaminen, Sonnenschutzmitteln, Retinoiden, antiallergenen Mitteln, Depigmentierungsmitteln, Entzündungshemmern, Anästhetika, Oberflächenmitteln, Feuchthaltemitteln, Abblätterungsmitteln, Emulgatoren, Stabilisatoren, Konservierungsmitteln, Antiseptika, Weichmachern, Verdickungsmitteln, Gleitmitteln, Benetzungsmitteln, Chelatbildnern, Duftstoffen, Farbstoffen und Hautdurchdringungsverstärkern.

15. Zusammensetzung nach Anspruch 1, wobei die Zusammensetzung einen pH-Wert von weniger als 7,0 aufweist.

16. Zusammensetzung nach Anspruch 15, wobei der pH-Wert etwa 3,5 bis etwa 7,0 beträgt.

17. Zusammensetzung nach Anspruch 16, wobei der pH-Wert etwa 3,5 bis etwa 4,0 beträgt.

18. Zusammensetzung nach Anspruch 2, wobei die Zusammensetzung etwa 1 bis etwa 50 Gew.% der Verbindung der Formel (I) umfasst und einen pH-Wert von weniger als 7,0 aufweist.

19. Zusammensetzung nach Anspruch 15, wobei das topische Vehikel 0,1 bis etwa 95 Gew.% der Verbindung der Formel (I); 0,5 bis etwa 50 Gew.% eines Weichmachers; und etwa 0,1 bis etwa 30 Gew.% eines Emulgators umfasst.

20. Zusammensetzung nach Anspruch 19, wobei die Verbindung ausgewählt ist aus der Gruppe, bestehend aus 3,6-Dioxaoctadisäure, 3,6,9-Trioxaundecandisäure, 3.,6,9,12-Tetraoxatetradecandisäure und 3,6,9,12,15-Pentaoxaheptadecandisäure; und wobei
der Weichmacher ausgewählt ist aus der Gruppe, bestehend aus Mineralöl, Rohvaseline, Paraffin, Ceresin, Ozokerit, mikrokristallinem Wachs, Perhydrosqualen, Dimethylpolysiloxanen, Methylphenylpolysiloxanen, Silikon-Glykol-Copolymeren, Triglyceridestern, acetylierten Monoglyceriden, ethoxylierten Glyceriden, Fettsäure-Alkylestern, Fettsäuren und -alkoholen, Lanolin und Lanolin-Derivaten, mehrwertigen Alkoholestern, Sterolen, Bienenwachsderivaten, mehrwertigen Alkoholen und Polyethem, und Amiden von Fettsäuren; und wobei
der Emulgator ausgewählt ist aus der Gruppe, bestehend aus Sorbitanen, alkoxylierten Fettalkoholen, Alkylpolyglykosiden, Seifen, Alkylsulfaten, Monoalkyl- und Dialkylphosphaten, Alkylsulfonaten und Acylisothionaten.

21. Zusammensetzung nach Anspruch 2, wobei die Zusammensetzung in einer Form ist, ausgewählt aus der Gruppe, bestehend aus einer Öl-in-wasser-Emulsion, einer Wasser-in-Öl-Emulsion, einer Wasser-in-Öl-in-Wasser-Emulsion und einer Öl-in-Wasser-in-Silikonflüssigkeit-Emulsion.

22. Zusammensetzung nach Anspruch 13, wobei das topische Vehikel einen Inhaltsstoff umfasst, ausgewählt aus der Gruppe, bestehend aus einem Ammoniumhydroxid, Cetearylalkohol/Ceteareth-20, EDTA, Glycerin, Glycerylmonostearat, Hydroxyethylcellulose, Imidazolidilyl-Harnstoff, Methylparaben, Myristylmyristat, Octylpalmitat und Propylenglycol und deren Gemischen.

23. Zusammensetzung nach Anspruch 13, umfassend etwa 0,1 Gew.% bis etwa 20 Gew.% 3,6,9-Trioxaundecansäure und ein Vehikel.

24. Zusammensetzung nach Anspruch 21, wobei das Vehikel umfasst:
(a) etwa 2 Gew.% bis etwa 10 Gew.% Glycerin;
(b) etwa 1 Gew.% bis etwa 10 Gew.% Propylenglykol;
(c) etwa 0,1 Gew.% bis etwa 2 Gew.% Hydroxyethylcellulose;
(d) etwa 0,1 Gew.% bis etwa 1 Gew.% Imidazolidilyl-Hamstoff;
(e) etwa 0,01 Gew.% bis etwa 2 Gew.% Dinatrium-EDTA; und
wobei die Zusammensetzung einen pH-Wert von etwa 7,0 oder weniger hat.

25. Zusammensetzung nach Anspruch 23, wobei das Vehikel umfasst:
(a) etwa 1 Gew.% bis etwa 10 Gew.% Glycerin;
(b) etwa 1 Gew.% bis etwa 10 Gew.% Propylenglykol;
(c) etwa 1 Gew.% bis etwa 10 Gew.% Octylpalmitat;
(d) etwa 1 Gew.% bis etwa 10 Gew.% Myristylmyristat;
(e) etwa 1 Gew.% bis etwa 6 Gew.% Cetearylalkohol/Ceteareth-20;
(f) etwa 0,5 Gew.% bis etwa 6 Gew.% Glycerylmonostearat;
(g) etwa 0,1 Gew.% bis etwa 2 Gew.% Hydroxyethylcellulose;
(h) etwa 0,1 Gew.% bis etwa 1 Gew.% Imidalozolidilyl-Harnstoff;
(i) etwa 0,05 Gew.% bis etwa 0,5 Gew.% Methylparaben;
(j) etwa 0,01 Gew.% bis etwa 2 Gew.% Dinatrium-EDTA; und wobei die Zusammensetzung einen pH-Wert von etwa 7,0 oder weniger hat.

26. Zusammensetzung nach Anspruch 23, wobei das Vehikel umfasst:
(a) etwa 2 Gew.% bis etwa 10 Gew.% Glycerin;
(b) etwa 1 Gew.% bis etwa 10 Gew.% Octylpalmitat;
(c) etwa 1 Gew.% bis etwa 10 Gew.% Myristylmyristat;
(d) etwa 1 Gew.% bis etwa 7 Gew.% Cetearylalkohol/Ceteareth-20;
(e) etwa 1 Gew.% bis etwa 10 Gew.% Propylenglykol;
(f) etwa 1 Gew.% bis etwa 6 Gew.% Glycerylmonostearat;
(g) etwa 0,1 Gew.% bis etwa 2 Gew.% Hydroxyethylcellulose;
(h) etwa 0,1 Gew.% bis etwa 1 Gew.% Imidazolidilyl-Harnstoff,
(i) etwa 0,05 Gew.% bis etwa 0,5 Gew.% Methylparaben;
(j) etwa 0,01 Gew.% bis etwa 2 Gew.% Dinatrium-EDTA; und
wobei die Zusammensetzung einen pH-Wert von etwa 7,0 oder weniger hat.

27. Verwendung einer wirksamen Menge der Verbindung der Formel (I): 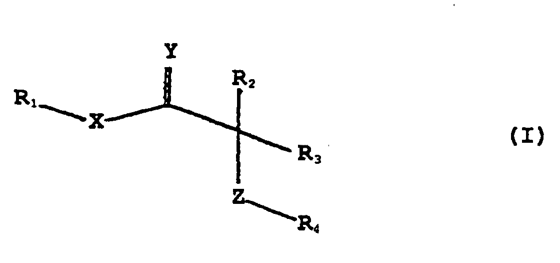 wobei R₄ (CR₅R₆-CR₇R₈-X₁)ₙ-CR₉R₁₀-C(=X₂)X₃R₁₁ ist, n eine ganze Zahl von 1 bis 18 ist; R₁, R₂, R₃, R₅, R₆, R₇, R₈, R₉, R₁₀ und R₁₁ unabhängig voneinander Wasserstoff oder Nicht-Wasserstoff Substituenten sind; und X, X₁, X₂, X₃, Y und Z unabhängig voneinander O, NH oder S sind, und wobei die Nicht-Wasserstoff-Substituenten ausgewählt sind aus der Gruppe bestehend aus Alkylen, Alkenylen, Oxaalkylen und Arylen, zur Herstellung eines Medikamentes zur Behandlung von Hautleiden, die durch eine anormale Abschuppung verursacht werden, begleitet werden oder verschlimmert werden.

28. Verwendung nach Anspruch 27, wobei die Verbindung mit einem geeigneten topischen Vehikel in einer topischen Zusammensetzung kombiniert wird.

29. Verwendung nach Anspruch 27, zudem umfassend das Aufbringen einer wirksamen Menge einers Gemischs von zwei oder mehreren verschiedenen Verbindungen der Formel (I) auf die Haut.

30. Verwendung nach Anspruch 28, wobei die Zusammensetzung etwa 0,1 bis etwa 95 Gew.% der Verbindung der Formel (I) umfasst.

31. Verwendung nach Anspruch 30, wobei die Zusammensetzung etwa 1 bis etwa 50 Gew.% der Verbindung der Formel (I) umfasst.

32. Verwendung nach Anspruch 30, wobei n eine ganze Zahl von 2 bis 12 ist.

33. Verwendung nach Anspruch 32, wobei die Nicht-Wasserstoff-Substituenten der Verbindung ausgewählt sind aus der Gruppe, bestehend aus Methyl, Ethyl, Propyl, Isopropyl, Butyl, Isobutyl, Hexyl, Heptyl, Octyl, Nonyl, Dodecanyl, Methoxy, Ethoxy, Propoxy, Butoxy, Cyclohexenyl, Hydroxymethyl, Hydroxyethyl, Hydroxypropyl, Cyclobutyl und Cyclohexanyl.

34. Verwendung nach Anspruch 27, wobei R₁, R₂, R₃, R₅, R₆, R₇, R₈, R₉, R₁₀, und R₁₁ jeweils Wasserstoff sind.

35. Verwendung nach Anspruch 27, wobei X, X₁, X₂, X₃, Y und Z jeweils Sauerstoff sind.

36. Verwendung nach Anspruch 27, wobei X, X₁, X₂, X₃, Y und Z jeweils Aminogruppen sind.

37. Verwendung nach Anspruch 27, wobei X, X₁, X₂, X₃, Y und Z jeweils Schwefel sind.

38. Verwendung nach Anspruch 27, wobei die Verbindung ausgewählt ist aus der Gruppe, bestehend aus 3,6-Dioxaoctadisäure; 3,6,9-Trioxaundecandisäure; 3,6,9,12-Tetraoxatetradecandisäure; 3,6,9,12,15-Pentaoxaheptadecandisäure; 2-Methyl-3,6,9-trioxaundecandisäure; 2-Ethyl-3,6,9,12-tetraoxatetradecandisäure; 2-Phenyl-3,6,9-trioxaundecandisäure; 3,6,9-Trioxaundecandisäurediethylester; 3,6,9-Triaminoundecandisäure; 3,6,9,12-Tetraminotetradecandisäure; 3-Amino-6,9-dioxaundecandisäure; 3,6-Diamino-9-oxaundecandisäure; 3,6,9-Trithioundecandisäure; 3,6-Dithio-9,12-dioxatetradecandisäure; 3-Amino-6,9-dioxaundecandisäuremonoamid; 3-Amino-6,9-dioxaundecandisäurediamid; 3,6,9-Trioxaundecandisäuremonoamid; 3,6,9-Trioxaundecandisäurediamid; 2,10-Dimethyl-3,6,9-trioxaundecandisäure; 2,10-Dimethyl-3,9-dithio-6-oxaundecandisäure; und deren Gemische.

39. Verwendung nach Anspruch 38, wobei die Verbindung 3,6,9-Trioxaundecansäure ist.

40. Verwendung nach Anspruch 28, wobei die Zusammensetzung zudem mindestens einen Wirkstoff, ausgewählt aus der Gruppe, bestehend aus Fungiziden, Vitaminen, Sonnenschutzmitteln, Retinoiden, antiallergenen Mitteln, Depigmentierungsmitteln, Entzündungshemmern, Anästhetika, Oberflächenmitteln, Feuchthaltemitteln, Abblätterungsmitteln, Emulgatoren, Stabilisatoren, Konservierungsmitteln, Antiseptika, Weichmachern, Verdickungsmitteln, Gleitmitteln, Benetzungsmitteln, Chelatbildnem, Duftstoffen, Farbstoffen und Hautdurchdringungsverstärkern umfasst.

41. Verwendung nach Anspruch 27, wobei die Hautbedingungen ausgewählt sind aus der Gruppe, bestehend aus trockener Haut, Ichtyose, palmaren und plantaren Hyperkeratosen, Kopfschuppen, Lichen simplex chronicus, Dariers-Erkrankung, Keratosen, Lentigines, Altersflecken, Melasmen, Haut mit Schönheitsfehlern, Akne, Psoriasis, Ekzeme, Pruritis, entzündlichen Dermatosen, Striae distensae, Warzen, Schwielen, Anzeichen von dermatologischem Altern, Hautrunzeln, Fältchen um den Mundbereich, unregelmäßiger Pigmentierung, Blässe, Verlust der Hautgeschmeidigkeit und Elastizität und Störungen bei Nägel, Kutikula und Haar.

42. Kosmetisches Verfahren zum Peeling von Haut, umfassend das Aufbringen einer Verbindung der Formel (I): 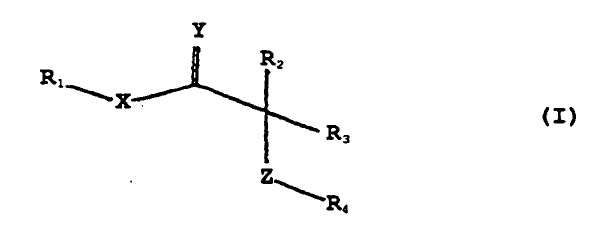 wobei R₄ (CR₅R₆-CR₇R₈-X₁)ₙ-CR₉R₁₀-C(=X₂)X₃R₁₁ist, n eine ganze Zahl von 1 bis 18 ist; R₁, R₂, R₃, R₅, R₆, R₇, R₈, R₉, R₁₀ und R₁₁ unabhängig voneinander Wasserstoff oder Nicht-Wasserstoff-Substituenten sind; und X, X₁, X₂, X₃, Y und Z unabhängig voneinander O, NH oder S sind, und wobei die Nicht-Wasserstoff-Substituenten ausgewählt sind aus der Gruppe bestehend aus Alkylen, Alkenylen, Oxaalkylen und Arylen, auf die Haut.

43. Verfahren zum Weichmachen von Haar, umfassend das Aufbringen einer Verbindung der Formel (I): 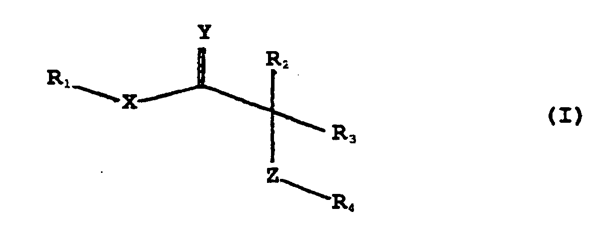 wobei R₄ (CR₅R₆-CR₇R₈-X₁)ₙ-CR₉R₁₀-C(=X₂)X₃R₁₁ ist, n eine ganze Zahl von 1 bis 18 ist; R₁, R₂, R₃, R₅, R₆, R₇, R₈, Rg, R₁₀ und R₁₁ unabhängig voneinander Wasserstoff oder Nicht-Wasserstoff-Substituenten sind; und X, X₁, X₂, X₃, Y und Z unabhängig voneinander O, NH oder S sind, und wobei die Nicht-Wasserstoff-Substituenten ausgewählt sind aus der Gruppe bestehend aus Alkylen, Alkenylen, Oxaalkylen und Arylen, auf das Haar.

## Revendications

1. Composition topique comprenant un véhicule topique approprié et un composé ayant la formule (I) : 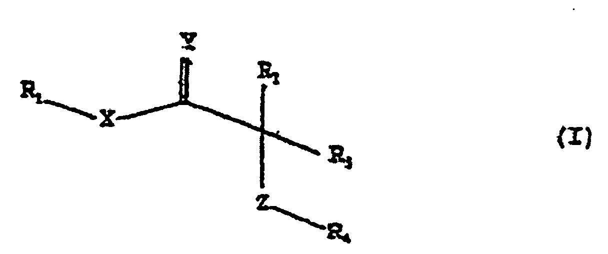 où R₄ est (CR₅R₆-CR₇R₈-X₁)ₙ -CR₉R₁₀-C(=X₂)X₃R₁₁, n est un nombre entier compris entre 1 et 18 ; R₁, R₂, R₃, R₅, R₆, R₇, R₈, R₉, R₁₀ et R₁₁ sont indépendamment des substituants d'hydrogène ou non ; et X, X₁, X₂, X₃, Y et Z sont indépendamment O, NH ou S ; et où les substituants qui ne sont pas de l'hydrogène sont sélectionnés dans le groupe consistant en alkyles, alkényles, oxa-alkyles et aryles.

2. Composition de la revendication 1, où ladite composition comprend un composé de la formule (I) avec entre environ 0,1 % et environ 95 % en poids de ladite composition.

3. Composition de la revendication 2, où ladite composition comprend un composé de la formule (I) avec entre environ 1 % et environ 50 % en poids de ladite composition.

4. Composition de la revendication 3, où ladite composition comprend un composé de la formule (I) avec entre environ 5 % et environ 20 % en poids de ladite composition.

5. Composition de la revendication 1, où n est un nombre entier compris entre 2 et 12.

6. Composition de la revendication 1, comprenant également un mélange d'au moins deux composés différents de la formule (I).

7. Composition de la revendication 1, où lesdits substituants qui ne sont pas de l'hydrogène sont sélectionnés dans le groupe consistant en méthyle, éthyle, propyle, isopropyle, butyle, isobutyle, hexyle, heptyle, octyle, nonyle, dodécanyle, méthoxy, éthoxy, propoxy, butoxy, cyclohexényle, hydroxyméthyle, hydroxyéthyle, hydroxypropyle, cyclobutyle et cyclohexanyle.

8. Composition de la revendication 1, où R₁, R₂, R₃, R₅, R₆, R₇, R₈, R₉, R₁₀ et R₁₁ sont tous de l'hydrogène.

9. Composition de la revendication 1, où X, X₁, X₂, X₃, Y et Z sont tous de l'oxygène.

10. Composition de la revendication 1, où X, X₁, X₂, X₃, Y et Z sont tous des groupes amino.

11. Composition de la revendication 1, où X, X₁, X₂, X₃, Y et Z sont tous du soufre.

12. Composition de la revendication 1, où ledit composé est sélectionné dans le groupe consistant en acide 3,6-dioxaoctadioïque ; acide 3,6,9-trioxaundécanédioïque ; acide 3,6,9,12-tétraoxatétradécanédioïque ; acide 3,6,9,12,5-pentaoxaheptadécanédioïque ; acide 2-méthyl-3,6,9-trioxaundécanédioqïque ; acide 2-éthyl-3,6,9,12-tétraoxatétradécanédioïque ; acide 2-phényl-3,6,9-trioxaundécanédioïque ; ester diéthylique de l'acide 3,6,9-trioxaundécanédioïque ; acide 3,6,9-triaminoundécanédioïque ; acide 3,6,9,12-tétraminotétradécanédioïque ; acide 3-amino-6,9-dioxaundécanédioïque; acide 3,6-diamino-9-oxaundécanédioïque ; acide 3,6,9-trithioundécanédioïque ; acide 3,6-dithio-9,12-dioxatétradécanédioïque ; monoamide de l'acide 3-amino-6,9-dioxaundécanédioqïque ; diamide de l'acide 3-amino-6,9-dioxaundécanédioïque ; monoamide de l'acide 3,6,9-trioxaundécanédioïque ; diamide de l'acide 3,6,9-trioxaundécanédioïque ; acide 2,10-diméthyl-3,6,9-trioxaundécanédioïque ; acide 2,10-diméthyl-3,9-dithio-6-oxaundécanédioïque ; et des mélanges de ces derniers.

13. Composition de la revendication 1, où ledit véhicule est sélectionné dans le groupe consistant en lotion, crème et gel.

14. Composition de la revendication 1, comprenant aussi au moins une substance active sélectionnée dans le groupe consistant en fongicides, vitamines, écrans solaires, rétinoïdes, agents antiallergéniques, agents de dépigmentation, agents anti-inflammatoires, anesthésiants, tensioactifs, agents hydratants, agents exfoliants, agents émulsifiants, stabilisateurs, agents conservateurs, antiseptiques, émollients, agents épaississants, lubrifiants, agents humectants, agents chélatants, parfums, colorants et agents facilitant la pénétration de la peau.

15. Composition de la revendication 1, où ladite composition a un pH de moins de 7,0.

16. Composition de la revendication 15, où ledit pH est compris entre environ 3,5 et environ 7,0.

17. Composition de la revendication 16, où ledit pH est compris entre environ 3,5 et environ 4,0.

18. Composition de la revendication 2, où ladite composition comprend entre environ 1 % et environ 50 % en poids dudit composé de la formule (I) et a un pH de moins de 7,0.

19. Composition de la revendication 15, où ledit véhicule topique comprend entre 0,1 % et environ 95 % en poids dudit composé de la formule (I) ; entre 0,5% et environ 50 % en poids d'un émollient ; et entre environ 0,1 % et environ 30 % en poids d'un agent émulsifiant.

20. Composition de la revendication 19, où ledit composé est sélectionné dans le groupe consistant en acide 3,6-dioxaoctadioïque, acide 3,6,9-trioxaundécanédioïque, acide 3,6,9,12-tétraoxatétradécanédioïque et acide 3,6,9,12,15-pentaoxaheptadécanédioïque ; et où
ledit émollient est sélectionné dans le groupe consistant en huile minérale, vaseline, paraffine, cérésine, ozocérite, cire microcrystalline, perhydrosqualène, polysiloxanes diméthyliques, polysiloxanes de méthyle-phényle, copolymères de silicone-glycol, esters de triglycérides, monoglycérides acétylés, glycérides éthoxylés, esters alkyliques d'acides gras, acides et alcools gras, lanoline et dérivés de lanoline, esters de polyalcools, stérols, dérivés de cire d'abeilles, polyéthers et polyalcools, et amides d'acides gras ; et où
ledit agent émulsifiant est sélectionné dans le groupe consistant en sorbitanes, alcools gras alkoxylés, polyglycosides alkyliques, savons, sulfates alkyliques, phosphates monoalkyliques et dialkyliques, sulfonates alkyliques et isothionates acyliques.

21. Composition de la revendication 2, où ladite composition a la forme sélectionnée dans le groupe consistant en, une émulsion huile dans l'eau, une émulsion eau dans l'huile, une émulsion eau dans l'huile dans l'eau et une émulsion huile dans l'eau dans silicone liquide.

22. Composition de la revendication 13, où ledit véhicule topique comprend un ingrédient sélectionné dans le groupe consistant en hydroxyde d'ammonium, alcool cétéarylique/Ceteareth-20, EDTA, glycérine, monostéarate de glycéryle, cellulose d'hydroxyéthyle, urée d'imidazolidilyle, méthyle paraben, mysistate, myristique, palmitate d'octyle, propylène glycol et des mélanges de ces substances.

23. Composition de la revendication 13, comprenant entre environ 0,1 % en poids et environ 20 % en poids d'acide 3,6,9-trioxaundécanoïque et un véhicule.

24. Composition de la revendication 21, où ledit véhicule comprend :
(a) entre environ 2 % en poids et environ 10 % en poids de glycérine ;
(b) entre environ 1 % en poids et environ 10 % en poids de propylène glycol ;
(c) entre environ 0,1 % en poids et environ 2 % en poids de cellulose d'hydroxyéthyle ;
(d) entre environ 0,1 % en poids et environ 1 % en poids d'urée d'imidazolidilyle ;
(e) entre environ 0,01 % en poids et environ 2 % en poids d'EDTA disodique ; et ladite composition a un pH d'environ 7,0 ou moins.

25. Composition de la revendication 23, où ledit véhicule comprend :
(a) entre environ 1 % en poids et environ 10 % en poids de glycérine ;
(b) entre environ 1 % en poids et environ 10 % en poids de propylène glycol ;
(c) entre environ 1 % en poids et environ 10 % en poids de palmitate d'octyle ;
(d) entre environ 1 % en poids et environ 10 % en poids de myristate myristique ;
(e) entre environ 1 % en poids et environ 6 % en poids d'alcool cétéarylique/Ceteareth-20 ;
(f) entre environ 0,5 % en poids et environ 6 % en poids de monostéarate de glycéryle ;
(g) entre environ 0,1 % en poids et environ 2 % en poids de cellulose d'hydroxyéthyle ;
(h) entre environ 0,1 % en poids et environ 1 % en poids d'urée d'imidazolidilyle ;
(i) entre environ 0,05 % en poids et environ 0,5 % en poids de méthyle paraben;
(j) entre environ 0,01 % en poids et environ 2 % en poids d'EDTA disodique ; et ladite composition a un pH d'environ 7,0 ou moins.

26. Composition de la revendication 23, où ledit véhicule comprend :
(a) entre environ 2 % en poids et environ 10 % en poids de glycérine ;
(b) entre environ 1 % en poids et environ 10 % en poids de palmitate d'octyle ;
(c) entre environ 1 % en poids et environ 10 % en poids de myristate myristique ;
(d) entre environ 1 % en poids et environ 7 % en poids d'alcool céréarylique/Ceteareth-20 ;
(e) entre environ 1 % en poids et environ 10 % en poids de propylène gtycol ;
(f) entre environ 1 % en poids et environ 6 % en poids de monostéarate de glycéryle ;
(g) entre environ 0,1 % en poids et environ 2 % en poids de cellulose d'hydroxyéthyle ;
(h) entre environ 0,1 % en poids et environ 1 % en poids d'urée d'imidazolidilyle ;
(i) entre environ 0,05 % en poids et environ 0,5 % en poids de méthyle paraben ;
(j) entre environ 0,01 % en poids et environ 2 % en poids d'EDTA disodique ; et ladite composition a un pH d'environ 7,0 ou moins.

27. Utilisation d'un montant effectif d'un composé de la formule (I) : 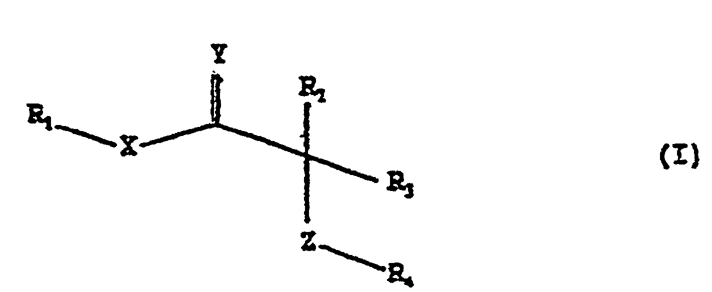 où R₄ est (CR₅R₆-CR₇R₈-X₁)ₙ -CR₉R₁₀ -C (=X₂) X₃R₁₁, n est un nombre entier compris entre 1 et 18 ; R₁, R₂, R₃, R₅, R₆, R₇, R₈, R₉, R₁₀ et R₁₁ sont indépendamment des substituants d'hydrogène ou non ; et X, X₁, X₂, X₃, Y et Z sont indépendamment O, NH ou S ; et où les substituants qui ne sont pas de l'hydrogène sont sélectionnés dans le groupe consistant en alkyles, alkényles, oxa-alkyles et aryles pour la fabrication de médicaments servant à traiter des pathologies cutanées causées, accompagnées ou exacerbées par une desquamation anormale.

28. Utilisation selon la revendication 27, où ledit composé est combiné à un véhicule topique approprié dans une composition topique.

29. Utilisation selon la revendication 27, comprenant également l'application à ladite peau d'un montant effectif d'un mélange d'au moins deux composés différents de la formule (I).

30. Utilisation selon la revendication 28, où ladite composition comprend entre environ 0,1 % et environ 95 % en poids dudit composé de la formule (I).

31. Utilisation selon la revendication 30, où ladite composition comprend entre environ 1 % et environ 50 % en poids dudit composé de la formule (I).

32. Utilisation selon la revendication 30, où n est un nombre entier compris entre 2 et 12.

33. Utilisation selon la revendication 32, où lesdits substituants qui ne sont pas de l'hydrogène dudit composé sont sélectionnés dans le groupe consistant en méthyle, éthyle, propyle, isopropyle, butyle, isobutyle, hexyle, heptyle, octyle, nonyle, dodécanyle, méthoxy, éthoxy, propoxy, butoxy, cyclohexényle, hydroxyméthyle, hydroxyéthyle, hydroxypropyle, cyclobutyle et cyclohexanyle.

34. Utilisation selon la revendication 27, où R₁, R₂, R₃, R₅, R₆, R₇, R₈, R₉, R₁₀ et R₁₁ sont tous de l'hydrogène.

35. Utilisation selon la revendication 27, où X, X₁, X₂, X₃, Y et Z sont tous de l'oxygène.

36. Utilisation selon la revendication 27, où X, X₁, X₂, X₃, Y et Z sont tous des groupes amino.

37. Utilisation selon la revendication 27, où X, X₁, X₂, X₃, Y et Z sont tous du soufre.

38. Utilisation selon la revendication 27, où ledit composé est sélectionné dans le groupe consistant en acide 3,6-dioxaoctadioïque ; acide 3,6,9-trioxaundécanédioïque ; acide 3,6,9,12-téiraoxatétradécanédioïque ; acide 3,6,9,12,15-pentaoxaheptadécanédioïque ; acide 2-méthyl-3,6,9-trioxaundécanédioqïque ; acide 2-éthyl-3,6,9,12-tétraoxatétradécanédioïque ; acide 2-phényl-3,6,9-trioxaundécanédioïque ; ester diéthylique de l'acide 3,6,9-trioxaundécanédioïque ; acide 3,6,9-triaminoundécanédioïque ; acide 3,6,9,12-tétraminotétradécanédioïque ; acide 3-amino-6,9-dioxaundécanédioïque ; acide 3,6-diamino-9-oxaundécanédioïque ; acide 3,6,9-trithioundécanédioïque ; acide 3,6-dithio-9,12-dioxatétradécanédioïque ; monoamide de l'acide 3-amino-6,9-dioxaundécanédioqïque ; diamide de l'acide 3-amino-6,9-dioxaundécanédioïque ; monoamide de l'acide 3,6,9-trioxaundécanédioïque ; diamide de l'acide-3,6,9-trioxaundécanédioïque; acide 2,10-diméthyl-3,6,9-trioxaundécanédioïque ; acide 2,10-diméthyl-3,9-dithio-6-oxaundécanédioïque ; et des mélanges de ces derniers.

39. Utilisation selon la revendication 38, où ledit composé est de l'acide 3,6,9-trioxaundécanoïque.

40. Utilisation selon la revendication 28, où ladite composition comprend également au moins une substance actif sélectionnée dans le groupe consistant en fongicides, vitamines, écrans solaires, rétinoïdes, agents antiallergéniques, agents de dépigmentation, agents anti-inflammatoires, anesthésiants, tensioactifs, agents hydratants, agents exfoliants, agents émulsifiants, stabilisateurs, agents conservateurs, antiseptiques, émollients, agents épaississants, lubrifiants, agents humectants, agents chélatants, parfums, colorants et agents facilitant la pénétration de la peau.

41. Utilisation selon la revendication 27, où lesdites pathologies cutanées sont sélectionnées dans le groupe consistant en peau sèche, ichtyose, hyperkératoses palmaires et plantaires, pellicules, névrodermite, maladie de Darier, kératoses, lentiginose, lentigo sénile, mélasmes, flétrissures cutanées, acné, psoriasis, eczéma, prurit, dermatoses inflammatoires, striae distensae, verrues, durillons, symptômes de vieillissement de la peau, rides, mini-rides autour de la bouche, pigmentation irrégulière, teint jaunâtre, perte d'élasticité et de résilience de la peau, et problèmes associés aux ongles, aux cuticules et aux cheveux.

42. Une méthode cosmétique pour peler la peau comprenant l'application à ladite peau d'un composé de la formule I : 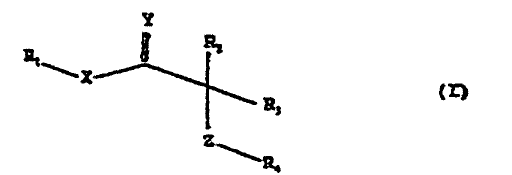 où R₄ est (CR₅R₆-CR₇R₈-X₁)ₙ -CR₉R₁₀-C(=X₂)X₃R₁₁, n est un nombre entier compris entre 1 et 18 ; R₁, R₂, R₃, R₅, R₆, R₇, R₈, R₉, R₁₀ et R₁₁ sont indépendamment des substituants d'hydrogène ou non ; et X, X₁, X₂, X₃, Y et Z sont indépendamment O, NH ou S ; et où les substituants qui ne sont pas de l'hydrogène sont sélectionnés dans le groupe consistant en alkyles, alkényles, oxa-alkyles et aryles.

43. Méthode d'assouplissement des cheveux comprenant l'application auxdits cheveux d'un composé de la formule I : 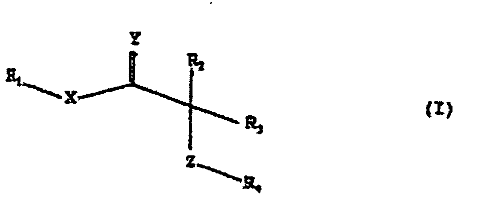 où R₄ est (CR₅R₆-CR₇R₈-X₁)ₙ -CR₉R₁₀-C(=X₂)X₃R₁₁, n est un nombre entier compris entre 1 et 18 ; R₁, R₂, R₃, R₅, R₆, R₇, R₈, R₉, R₁₀ et R₁₁ sont indépendamment des substituants d'hydrogène ou non ; et X, X₁, X₂, X₃, Y et Z sont indépendamment O, NH ou S ; et où les substituants qui ne sont pas de l'hydrogène sont sélectionnés dans le groupe consistant en alkyles, alkényles, oxa-alkyles et aryles.
